# EUROPEAN PATENT SPECIFICATION

(11) **EP 1 509 593 B1**
(45) Date of publication and mention of the grant of the patent: **15.08.2007**
(21) Application number: 03757462.1
(22) Date of filing: 05.06.2003
(51) Int. Cl.: C12M 1/34, C12M 3/00, G01N 33/543, G01N 33/487, B01J 19/00, G01N 21/01

(54) **METHOD COMPRISING REACTOR SYSTEMS HAVING A LIGHT-INTERACTING COMPONENT**
VERFAHREN ENHALTEND REAKTOREN MIT EINEM BELEUCHTETEN BAUELEMENT
PROCEDES COMPRENANT DES SYSTEMES REACTEURS POURVUS D'UN COMPOSANT INTERAGISSANT AVEC LA LUMIERE

(30) Priority: 05.06.2002 US 386322 P
(43) Date of publication of application: 02.03.2005
(73) Proprietor: Bioprocessors Corporation, Woburn, MA 01801 (US)
(72) Inventor: MILLER, Scott, Somerville, MA 02144 (US); LEBLANC, Sean, J., Westminister, MA 01473 (US); RODGERS, Seth, T., Somerville, MA 02144 (US); ZARUR, Andrey, J., Winchester, MA 01890 (US)
(74) Representative: Killin, Stephen James
(86) International application number: PCT/US2003/018240
(87) International publication number: WO 2003/104384

(56) References cited:
- WO-A-00/43552
- WO-A-01/07891
- WO-A-01/92870
- WO-A-99/57310
- WO-A-03/035824
- US-B1- 6 377 721

## Description

### BACKGROUND

### Field of the Invention

This invention relates to components that interact with light and, in particular, to components for use in reactors that interact with light.

### Description of the Related Art

A wide variety of reaction systems are known for the production of products of chemical and/or biochemical reactions. Chemical plants involving catalysis, biochemical fermenters, pharmaceutical production plants, and a host of other systems are well-known. Biochemical processing may involve the use of a live microorganism (e.g., cells) to produce a substance of interest.

Cells are cultured for a variety of reasons. Increasingly, cells are cultured for proteins or other valuable materials they produce. Many cells require specific conditions, such as a controlled environment. The presence of nutrients, metabolic gases such as oxygen and/or carbon dioxide, humidity, as well as other factors such as temperature, may affect cell growth. Cells require time to grow, during which favorable conditions must be maintained. In some cases, such as with particular bacterial cells, a successful cell culture may be performed in as little as 24 hours. In other cases, such as with particular mammalian cells, a successful culture may require about 30 days or more.

Typically, cell cultures are performed in media suitable for cell growth and containing necessary nutrients. The cells are generally cultured in a location, such as an incubator, where the environmental conditions can be controlled. Incubators traditionally range in size from small incubators (e.g., about 1 cubic foot) for a few cultures up to an entire room or rooms where the desired environmental conditions can be carefully maintained.

Recently, as described in International Patent Application Serial No. PCT/LTS01/07679, published on September 20, 2001 as WO O1/68257, entitled "Microreactors," cells have also been cultured on a very small scale (i.e., on the order of a few milliliters or less), so that, among other things, many cultures can be performed in parallel.

### SUMMARY OF THE INVENTION

This invention generally relates to light-interacting components for use in reactors and reactor systems. A variety of reactors and reactor systems are provided as well as methods involving the interaction of light with various materials. The subject matter of this application involves, in some cases, interrelated products and/or uses, alternative solutions to a particular problem, and/or a plurality of different uses of a single system or article.

In one aspect, the invention includes an apparatus as part of a method. In one set of embodiments, the apparatus includes a chip comprising a predetermined reaction site having a volume of less than about 1 ml, the predetermined reaction site constructed and arranged to maintain at least one living cell at the site, and at least one waveguide in optical communication with the predetermined reaction site. The apparatus, in another set of embodiments, includes a chip comprising a predetermined reaction site having at least one substantially hydrophobic surface and a volume of less than about 1 ml, and at least one waveguide in optical communication with the predetermined reaction site. In yet another set of embodiments, the apparatus includes a chip comprising a predetermined reaction site having at least one substantially hydrophilic surface and a volume of less than about 1 ml, and at least one waveguide in optical communication with the predetermined reaction site. In still another set of embodiments, the apparatus includes a chip comprising a predetermined reaction site having at least one substantially cytophilic surface and a volume of less than about 1 ml, and at least one waveguide in optical communication with the predetermined reaction site. The apparatus, in yet another set of embodiments, includes a chip comprising a predetermined reaction site having at least one substantially cytophobic surface and a volume of less than about 1 ml; and at least one waveguide in optical communication with the predetermined reaction site.

In one set of embodiments, the apparatus is defined, at least in part, by a chip comprising a predetermined reaction site having a volume of less than about 1 ml, and a milled waveguide in optical communication with the predetermined reaction site. The apparatus includes, in another set of embodiments, a chip comprising a predetermined reaction site having a volume of less than about 1 ml; and a machined waveguide in optical communication with the predetermined reaction site.

The apparatus, in some embodiments, includes a chip comprising a predetermined reaction site having a volume of less than about 1 ml, the predetermined reaction site constructed and arranged to maintain at least one living cell at the site, and an optical element in optical communication with the predetermined reaction site.

In one set of embodiments, the apparatus includes a chip comprising a predetermined reaction site having a volume of less than about 1 ml, the predetermined reaction site constructed and arranged to maintain at least one living cell at the site, and a photodetector in optical communication with the predetermined reaction site. The apparatus, in another set of embodiments, includes a predetermined reaction site having a volume of less than about 1 ml, the predetermined reaction site constructed and arranged to maintain at least one living cell at the site, and a source of light in optical communication and integrally connected with the predetermined reaction site.

The apparatus, in one set of embodiments, includes a chip comprising a predetermined reaction site having a volume of less than about 1 ml, the predetermined reaction site constructed and arranged to maintain at least one living cell at the site, and a filter able to filter light entering or exiting the predetermined reaction site, where the filter is integrally connected to the chip. In another set of embodiments, the apparatus includes a chip comprising a predetermined reaction site having a volume of less than about 1 ml, the predetermined reaction site constructed and arranged to maintain at least one living cell at the site, and a light-interacting component integrally connected to the predetermined reaction site. In yet another set of embodiments, the invention includes a chip comprising a predetermined reaction site having a volume of less than about 1 ml, and an actuator able to target a first cell type within the predetermined reaction site without targeting a second cell type. The apparatus, in certain embodiments, comprises a chip comprising a predetermined reaction site having an inlet, an outlet, and a volume of less than about 1 ml, the predetermined reaction site constructed and arranged to maintain at least one living cell at the site, where the chip is substantially transparent.

In one set of embodiments, the apparatus includes a chip comprising a predetermined reaction site having at least one substantially hydrophilic surface and a volume of less than about 1 ml; and a sensor. The sensor can be, in some cases, a sensor for measuring one or more of temperature, pressure, relative humidity, pH, shear stress, or osmolarity.

In another set of embodiments, the apparatus includes a chip comprising a predetermined reaction site having a volume of less than about 1 ml, and an actuator able to target a first cell type within the predetermined reaction site without targeting a second cell type. In yet another set of embodiments, the apparatus includes a chip comprising a reactor comprising a predetermined reaction site having an inlet, an outlet, and a volume of less than about 1 ml, the predetermined reaction site constructed and arranged to maintain at least one living cell at the site, where the reactor is substantially transparent.

In another aspect, the invention includes a method comprising the steps of providing a predetermined reaction site having a volume of less than about 1 ml, the predetermined reaction site constructed and arranged to maintain at least one living cell at the site, providing material in the predetermined reaction site, the material having a smallest dimension, directing electromagnetic radiation having an average beam diameter less than the smallest dimension of the material, allowing the electromagnetic radiation to interact with the material to produce altered radiation, and determining the altered radiation.

The method, in another set of embodiments, includes the steps of providing a predetermined reaction site having a volume of less than about 1 ml, the predetermined reaction site constructed and arranged to maintain at least one living cell at the site, and optically causing a biological change in a biological material located at the predetermined reaction site. The method, in still another set of embodiments, includes the steps of providing a chip having a predetermined reaction site having a volume of less than about 1 ml, the predetermined reaction site constructed and arranged to maintain at least one living cell at the site, providing material in the predetermined reaction site, directing light from a source within the chip at the material, and producing an image of the material. In one set of embodiments, the method includes the steps of providing a chip comprising a predetermined reaction site having an inlet, an outlet, and a volume of less than about 1 ml, the predetermined reaction site constructed and arranged to maintain at least one living cell at the site, and optically addressing the predetermined reaction site.

In one embodiment, the method includes the steps of providing a reactor comprising a predetermined reaction site having an inlet, an outlet, and a volume of less than about 1 ml, the predetermined reaction site constructed and arranged to maintain at least one living cell at the site, and optically addressing the predetermined reaction site.

In another aspect, the invention is directed to a method of making a chip and/or a reactor system, e.g., as described in any of the embodiments herein. In yet another aspect, the invention is directed to a method of using a chip and/or a reactor system, e.g., as described in any of the embodiments described herein.

Other advantages and novel features of the invention will become apparent from the following detailed description of various non-limiting embodiments of the invention when considered in conjunction with the accompanying drawings, which are schematic and are not intended to be drawn to scale. In the figures, each identical or nearly identical component illustrated is typically represented by a single numeral. For the purposes of clarity, not every component is labeled in every figure, nor is every component of each embodiment of the invention shown where illustration is not necessary to allow those of ordinary skill in the art to understand the invention. In cases where the present specification and a document incorporated by reference include conflicting disclosure, the present specification shall control.

### BRIEF DESCRIPTION OF THE DRAWINGS

Non-limiting embodiments of the present invention will be described by way of example with reference to the accompanying drawings in which:
Fig. 1 illustrates one embodiment of the invention, showing light interacting with a reaction site;
Fig. 2 illustrates the detection of light from a reaction site in another embodiment of the invention;
Fig. 3 illustrates another embodiment of the invention having a waveguide;
Fig. 4 illustrates another embodiment of the invention having an optical element;
Fig. 5 illustrates another embodiment of the invention having a collimating optical element;
Fig. 6 illustrates another embodiment of the invention having a component able to convert light to electricity;
Fig. 7 illustrates another embodiment of the invention having a sample able to produce light;
Fig. 8 illustrates another embodiment of the invention having more than one site;
Fig. 9 illustrates another embodiment of the invention, a portion of which is rotatable;
Fig. 10 illustrates another embodiment of the invention, having more than one site;
Fig. 11 is a graph of intensity (in relative units) versus relative concentration, in an embodiment of the invention;
Fig. 12 is a graph of optical density at 480 nm versus time in an experiment using an embodiment of the invention; and
Fig. 13 is a graph of pH versus relative intensity, in accordance with one embodiment of the invention.

### DETAILED DESCRIPTION

Various aspects of the present invention relate to light-interacting components suitable for use in chips and other reactor systems. These components may include waveguides, optical fibers, light sources, photodetectors, optical elements, and the like. If waveguides are used, they may be fashioned out of any material able to transmit light to or from the reaction site. The chip may contain a reaction site having a volume of less than about 1 ml. In some embodiments, the chip may be constructed in such a way as to be able to support a living cell. The chip may be used for imaging or analysis, or the chip may be used to facilitate a chemical or biological reaction, which may be light-sensitive or light-activated in certain cases. Other facilitated reactions may include the production or consumption of a chemical or biological species. In some embodiments, the chip may include more than one component or component type, or more than one reaction site.

The following prior art documents are cited: International Patent Publication No. WO 01/68257, published September 20, 2001, entitled "Microreactor," by Jury, *et al.;* U.S. Patent Publication No. 2003/0077817, published April 24, 2003, entitled "Microfermentor Device and Cell Based Screening Method," by A. Zarur, et *al..*

The present invention generally relates to reactors and chips, as part of a method particularly microfluidic reactors and chips, having the ability to measure and/or control environmental factors therein. A chip may include one or more reactors, each of which may include one or more reaction sites, and these terms are defined more fully below. In one embodiment, the chip is able to detect and/or measure an environmental factor associated with a reactor or a reaction site of a reactor on the chip, such as the temperature, pressure, CO₂ concentration, O₂ concentration, relative humidity, pH, or a combination of any of these, for example, by using one or more sensors positioned in sensing communication with the reaction site.

A "chip," as used herein, is an integral article that includes one or more reactors. "Integral article" means a single piece of material, or assembly of components integrally connected with each other. As used herein, the term "integrally connected," when referring to two or more objects, means objects that do not become separated from each other during the course of normal use, e.g., cannot be separated manually; separation requires at least the use of tools, and/or by causing damage to at least one of the components, for example, by breaking, peeling, etc. (separating components fastened together via adhesives, tools, etc.).
A chip can be connected to or inserted into a larger framework defining an overall reaction system. The system can be defined primarily by other chips, chassis, cartridges, cassettes, and/or by a larger machine or set of conduits or channels, sources of reactants, cells, and/or nutrients, outlets, sensors, actuators, and/or controllers. Typically, the chip can be a generally flat or planar article (i.e., having one dimension that is relatively small compared to the other dimensions); however, in some cases, the chip can be a non-planar article, for example, the chip may have a cubical shape, a solid or block shape, etc.

As used herein, a "membrane" is a three-dimensional material having any shape such that one of the dimensions is substantially smaller than the other dimensions. In some cases, the membrane may be generally flexible or non-rigid. As an example, a membrane may be a rectangular or circular material with a length and width on the order of millimeters, centimeters, or more, and a thickness of less than a millimeter, and in some cases, less than 100 microns, less than 10 microns, or less than 1 micron. Some membranes are semipermeable membranes, which those of ordinary skill in the art will recognize to be membranes permeable with respect to at least one species, but not readily permeable with respect to at least one other species. For example, a semipermeable membrane may allow oxygen to permeate across it, but not allow water vapor to do so, or allows water vapor to permeate it, but at a permeability that is at least an order of magnitude less. Or a semipermeable membrane may be selected to allow water to permeate across it, but not certain ions. Some membranes are transparent to particular light (e.g. infrared, UV, or visible light; light of a wavelength with which a device utilizing the membrane interacts; visible light if not otherwise indicted). Where a membrane is "substantially transparent," it absorbs no more than 50% of light, or in other embodiments no more than 25% or 10% of light, as described more fully herein. In some cases, a membrane may be both semipermeable and substantially transparent.

As used herein, a "reactor" is the combination of components including a reaction site, any chambers (including reaction chambers and ancillary chambers), channels, ports, inlets and/or outlets (i.e., leading to or from a reaction site), sensors, actuators, membranes, and the like, which, together, operate to promote and/or monitor a biological, chemical, or biochemical reaction, interaction, operation, or experiment at a reaction site, and which can be part of a chip. Examples of reactors include chemical or biological reactors and cell culturing devices, as well as the reactors described in International Patent Application Serial No. PCT/USO1/07679, published on September 20, 2001 as WO 01/68257. Reactors can include one or more reaction sites or chambers. The reactor may be used for any chemical, biochemical, and/or biological purpose, for example, cell growth, pharmaceutical production, chemical synthesis, hazardous chemical production, drug screening, materials screening, drug development, chemical remediation of warfare reagents, or the like. In one set of embodiments, a reactor of the invention comprises a matrix or substrate of a few millimeters to centimeters in size, containing channels with dimensions on the order of, e.g., tens or hundreds of micrometers. Reagents of interest may be allowed to flow through these channels, for example to a reaction site, or between different reaction sites, and the reagents may be mixed or reacted in some fashion. The products of such reactions can be recovered, separated, and treated within the system in certain cases.

As used herein, a "reaction site" is defined as a site within a reactor that is constructed and arranged to produce a physical, chemical, biochemical, and/or biological reaction during use of the reactor. More than one reaction site may be present within a reactor or a chip in some cases, for example, at least 5 reaction sites, at least 7 reaction sites, at least 10 reaction sites, at least 20 reaction sites, at least 30 reaction sites, or at least 50 reaction sites or more may be present within a reactor or a chip. The reaction site may be defined as a region where a reaction is allowed to occur; for example, the reactor may be constructed and arranged to cause a reaction within a channel, one or more chambers, at the intersection of two or more channels, etc. The reaction may be, for example, a mixing or a separation process, a reaction between two or more chemicals, a light-activated or a light-inhibited reaction, a biological process, and the like. In some embodiments, the reaction may involve an interaction with light that does not lead to a chemical change, for example, a photon of light may be absorbed by a substance associated with the reaction site and converted into heat energy or re-emitted as fluorescence. In certain embodiments, the reaction site may also include one or more cells. Thus, in some cases, the reaction site may be defined as a region surrounding a location where cells are to be placed within the reactor, for example, a cytophilic region within the reactor.

Many embodiments and arrangements of the invention are described with reference to a chip, or to a reactor, and those of ordinary skill in the art will recognize that the invention can apply to either or both. For example, a channel arrangement may be described in the context of one, but it will be recognized that the arrangement can apply in the context of the other (or, typically, both: a reactor which is part of a chip). It is to be understood that all descriptions herein that are given in the context of a reactor or chip apply to the other, unless inconsistent with the description of the arrangement in the context of the definitions of "chip" and "reactor" herein.

In some embodiments, the reaction site may be defined by geometrical considerations. For example, the reaction site may be defined as a chamber in a reactor, a channel, an intersection of two or more channels, or other location defined in some fashion (e.g., formed or etched within a substrate that can define a reactor and/or chip). Other methods of defining a reaction site are also possible. In some embodiments, the reaction site may be artificially created, for example, by the intersection or union of two or more fluids (e.g., within one or several channels), or by constraining a fluid on a surface, for example, using bumps or ridges on the surface to constrain fluid flow. In other embodiments, the reaction site may be defined through electrical, magnetic, and/or optical systems. For example, a reaction site may be defined as the intersection between a beam of light and a fluid channel.

The volume of the reaction site can be very small in certain embodiments. Specifically, the reaction site may have a volume of less than one liter, less than about 100 ml, les than about 10 ml, less than about 5 ml, less than about 3 ml, less than about 2 ml, less than about 1 ml, less than about 300 microliters, less than about 100 microliters, less than about 30 microliters, or less than about 10 microliters in various embodiments. The reaction site may also have a volume of less than about 5 microliters, or less than about 1 microliter in certain cases.

In some cases, cells can be present at the reaction site, and sensor(s) associated with the chip or reactor may be able to determine the number of cells, the density of cells, the status or health of the cell, the cell type, the physiology of the cells, etc. In certain cases, the reactor can also maintain or control one or more environmental factors associated with the reaction site, for example, in such a way as to support a chemical reaction or a living cell. In one set of embodiments, a sensor may be connected to an actuator and/or a microprocessor able to produce an appropriate change in an environmental factor within the reaction site. The actuator may be connected to an external pump, the actuator may cause the release of a substance from a reservoir, or the actuator may produce sonic or electromagnetic energy to heat the reaction site or selectively kill a type of cell susceptible to that energy. The reactor can include one or more than one reaction site, and one or more than one sensor, actuator, processor, and/or control system associated with the reaction site(s). It is to be understood that any reaction site or a sensor technique disclosed herein can be provided in combination with any combination of other reaction sites and sensors.

As used herein, a "channel" is a conduit associated with a reactor (within, leading to, or leading from a reaction site) that is able to transport one or more fluids specifically from one location to another, for example, from an inlet of the reactor to a reaction site, as further described below. The channel may be a closed channel, or a channel that is open, for example, open to the external environment surrounding the reactor or chip containing the reactor. The channel can include characteristics that facilitate control over fluid transport, e.g., structural characteristics (e.g., an elongated indentation), physical/chemical characteristics (e.g., hydrophobicity vs. hydrophilicity) and/or other characteristics that can exert a force (e.g., a containing force) on a fluid when within the channel. The fluid within the channel may partially or completely fill the channel. In some cases the fluid may be held or confined within the channel or a portion of the channel in some fashion, for example, using surface tension (i.e., such that the fluid is held within the channel within a meniscus, such as a concave or convex meniscus). The channel may have any suitable cross-sectional shape that allows for fluid transport, for example, a square channel, a circular channel, a rounded channel, a rectangular channel (e.g., having any aspect ratio), a triangular channel, an irregular channel, etc. The channel may be of any size within the reactor or chip. For example, the channel may have a largest dimension perpendicular to a direction of fluid flow within the channel of less than about 1000 micrometers in some cases, less than about 500 micrometers in other cases, less than about 200 micrometers in still other cases, less than about 100 micrometers in still other cases, or less than about 50 or 25 micrometers in still other cases. In some embodiments, the dimensions of the channel may be chosen such that fluid is able to freely flow through the channel, for example, if the fluid contains cells. The dimensions of the channel may also be chosen in certain cases, for example, to allow a certain volumetric or linear flowrate of fluid within the channel. Of course, the number of channels, the shape or geometry of the channels, and the placement of channels within the chip can be determined by those of ordinary skill in the art.

Chips as part of a method of the invention may also include a plurality of inlets and/or outlets that can receive and/or output any of a variety of reactants, products, and/or fluids, for example, directed towards one or more reactors and/or reaction sites. At least a portion of the plurality of inlets and/or outlets may be in fluid communication with one or more reaction sites within the chip. In some cases, the inlets and/or outlets may also contain one or more sensors and/or actuators, as further described below. Essentially, the chip may have any number of inlets and/or outlets from one to tens of hundreds that can be in fluid communication with one or more reactors and/or reaction sites. Less than 5 or 10 inlets and/or outlets may be provided to the reactor and/or reaction site(s) for certain reactions, such as biological or biochemical reactions. In some cases each reactor may have around 25 inlets and/or outlets, in other cases around 50 inlets and/or outlets, in still other cases around 75 inlets and/or outlets, or around 100 or more inlets and/or outlets in still other cases.

As one example, the inlets and/or outlets of the chip, directed to one or more reactors and/or reaction sites may include inlets and/or outlets for a fluid such as a gas or a liquid, for example, for a waste stream, a reactant stream, a product stream, an inert stream, etc. In some cases, the chip may be constructed and arranged such that fluids entering or leaving reactors and/or reaction sites do not substantially disturb reactions that may be occurring therein. For example, fluids may enter and/or leave a reaction site without affecting the rate of reaction in a chemical, biochemical, and/or biological reaction occurring within the reaction site, or without disturbing and/or disrupting cells that may be present within the reaction site. Examples of inlet and/or outlet gases may include, but are not limited to, CO₂, CO, oxygen, hydrogen, NO, NO₂ water vapor, nitrogen, ammonia, acetic acid, etc. As another example, an inlet and/or outlet fluid may include liquids and/or other substances contained therein, for example, water, saline, cells, cell culture medium, blood or other bodily fluids, antibodies, pH buffers, solvents, hormones, carbohydrates, nutrients, growth factors, antifoaming agents (e.g., to prevent production of foam and bubbles), proteins, antibodies, and the like. The inlet and/or outlet fluid may also include a metabolite in some cases. A "metabolite," as used herein, is any molecule that can be metabolized by a cell. For example, a metabolite may be or include an energy source such as a carbohydrate or a sugar, for example, glucose, fructose, galactose, starch, corn syrup, and the like. Other example metabolites include hormones, enzymes, proteins, signaling peptides, amino acids, etc.

The inlets and/or outlets may be formed within the chip by any suitable technique known to those of ordinary skill in the art, for example, by holes or apertures that are punched, drilled, molded, milled, etc. within the chip or within a portion of the chip, such as a substrate layer. In some cases, the inlets and/or outlets may be lined, for example, with an elastomeric material. In certain embodiments, the inlets and/or outlets may be constructed using self-sealing materials that may be re-usable in some cases. For example, an inlet and/or outlet may be constructed out of a material that allows the inlet and/or outlet to be liquid-tight (i.e., the inlet and/or outlet will not allow a liquid to pass therethrough without the application of an external driving force, but may admit the insertion of a needle or other mechanical device able to penetrate the material under certain conditions). In some cases, upon removal of the needle or other mechanical device, the material may be able to regain its liquid-tight properties (i.e., a "self-sealing" material). Non-limiting examples of self-sealing materials suitable for use with the invention include, for example, polymers such as polydimethylsiloxane ("PDMS"), natural rubber, HDPE, or silicone materials such as Formulations RTV 108, RTV 615, or RTV 118 (General Electric, New York, NY).

In some embodiments, the chip of the present invention may include very small elements, for example, sub-millimeter or microfluidic elements. For example, in some embodiments, the chip may include at least one reaction site having a cross sectional dimension of no greater than, for example, 100 mm, 80 mm, 50 mm, or 10 mm. In some embodiments, the reaction site may have a maximum cross section no greater than, for example, 100 mm, 80 mm, 50 mm, or 10 mm. As used herein, the "cross section" refers to a distance measured between two opposed boundaries of the reaction site, and the "maximum cross section" refers to the largest distance between two opposed boundaries that may be measured. In other embodiments, a cross section or a maximum cross section of a reaction site may be less than 5 mm, less than 2 mm, less than 1 mm, less than 500 micrometers, less than 300 micrometers, less than 100 micrometers, less than 10 micrometers, or less than 1 micrometer or smaller. As used herein, a "microfluidic chip" is a chip comprising at least one fluidic element having a sub-millimeter cross section, i.e., having a cross section that is less than 1 mm. As one particular non-limiting example, a reaction site may have a generally rectangular shape, with a length of 80 mm, a width of 10 mm, and a depth of 5 mm.

While one reaction site may be able to hold and/or react a small volume of fluid as described herein, the technology associated with the invention also allows for scalability and parallelization. With regard to throughput, an array of many reactors and/or reaction sites within a chip, or within a plurality of chips, can be built in parallel to generate larger capacities. Additionally, an advantage may be obtained by maintaining production capacity at the small scale of reactions typically performed in the laboratory, with scale-up via parallelization. It is a feature of the invention that many reaction sites may be arranged in parallel within a reactor of a chip and/or within a plurality of chips. Specifically, at least five reaction sites can be constructed to operate in parallel, or in other cases at least about 7, about 10, about 50, about 100, about 500, about 1,000, about 5,000, about 10,000, about 50,000, or even about 100,000 or more reaction sites can be constructed to operate in parallel. In some cases, the number of reaction sites may be selected so as to produce a certain quantity of a species or product, or so as to be able to process a certain amount of reactant. Of course, the exact locations and arrangement of the reaction site(s) within the reactor or chip will be a function of the specific application.

Additionally, any embodiment described herein can be used in conjunction with a collection chamber connectable ultimately to an outlet of one or more reactors and/or reaction sites of a chip. The collection chamber may have a volume of greater than 10 milliliters or 100 milliliters in some cases. The collection chamber, in other cases, may have a volume of greater than 100 liters or 500 liters, or greater than 1 liter, 2 liters, 5 liters, or 10 liters. Large volumes may be appropriate where the reactors and/or reaction sites are arranged in parallel within one or more chips, e.g., a plurality of reactors and/or reaction sites may be able to deliver a product to a collection chamber.

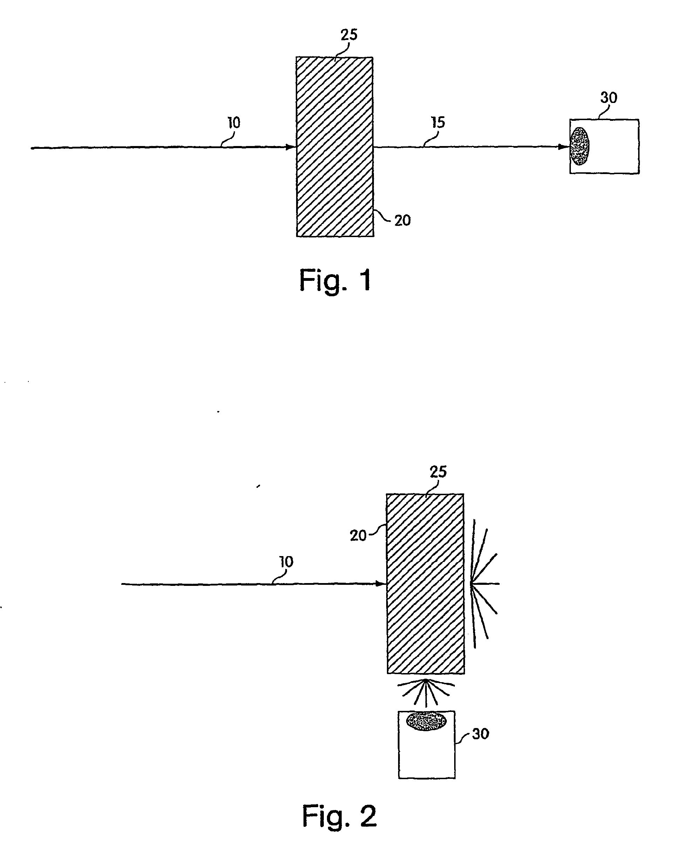

In some embodiments, the reaction site(s) and/or the channels in fluidic communication with the reaction site(s) are free of active mixing elements. In these embodiments, the reactor of the chip can be constructed in such a way as to cause turbulence in the fluids provided through the inlets and/or outlets, thereby mixing and/or delivering a mixture of the fluids, preferably without active mixing, where mixing is desired. Specifically, the reactor and/or reaction site(s) may include a plurality of obstructions in the flow path of the fluid that causes fluid flowing through the flow path to mix, for example, as shown in mixing unit 12 in Fig. 1. These obstructions can be of essentially any geometrical arrangement for example, a series of pillars. As used herein, "active mixing elements" is meant to define mixing elements such as blades, stirrers, or the like, which are movable relative to the reaction site(s) and/or channels themselves, that is, movable relative to portion(s) of the reactor defining a reaction site a or a channel.

The term "determining," as used herein, generally refers to the measurement and/or analysis of a substance (e.g., within a reaction site), for example, quantitatively or qualitatively, or the detection of the presence or absence of the substance. "Determining" may also refer to the measurement and/or analysis of an interaction between two or more substances, for example, quantitatively or qualitatively, or by detecting the presence or absence of the interaction. Examples of techniques suitable for use in the invention include, but are not limited to, gravimetric analysis, calorimetry, pressure or temperature measurement, spectroscopy such as infrared, absorption, fluorescence, UV/visible, FTIR ("Fourier Transform Infrared Spectroscopy"), or Raman; gravimetric techniques; ellipsometry; piezoelectric measurements; immunoassays; electrochemical measurements; optical measurements such as optical density measurements; circular dichroism; light scattering measurements such as quasielectric light scattering; polarimetry; refractometry; or turbidity measurements, including nephelometry.

Chips of the invention can be constructed and arranged such that they are able to be stacked in a predetermined, prealigned relationship with other, similar chips, such that the chips are all oriented in a predetermined way (e.g., all oriented in the same way) when stacked together. When a chip of the invention is designed to be stacked with other, similar chips, it often can be constructed and arranged such that at least a portion of the chip, such as a reaction site, is in fluidic communication with one or more of the other chips and/or reaction sites within other chips. This arrangement can find use in parallelization of chips, as discussed herein.

In one set of embodiments, the chip is constructed and arranged such that the chip is able to be stably connected to a microplate. As used herein, "stably connected" refers to systems in which two components are connected such that a specific motion or force is necessary to disconnect the two components from each other, i.e., the two components cannot be dislodged by random vibration or displacement (e.g., accidentally lightly bumping a component). The components can be stably connected by way of pegs, screws, snap-fit components, matching sets of indentations and protrusions, or the like. A "microplate" is also sometimes referred to as a "microtiter" plate, a "microwell" plate, or other similar terms known to the art. The microplate may include any number of wells. For example, as is typically used commercially, the microplate may be a six-well microplate, a 24-well microplate, a 96-well microplate, a 384-well microplate, or a 1,536-well microplate. The wells may be of any suitable shape, for example, cylindrical or rectangular. The microplate may also have other numbers of wells and/or other well geometries or configurations, for instance, in certain specialized applications.

In another set of embodiments, one or more reaction sites may be positioned in association with a chip such that, when the chip is stably connected to other chips and/or microplates, one or more reaction sites of the chip are positioned or aligned to be in chemical, biological, or biochemical communication with, or chemically, biologically, or biochemically connectable with one or more reaction sites of the other chip(s) and/or one or more wells of the microplate(s). "Alignment," in this context, can mean complete alignment, such that the entire area of the side of a reaction site adjacent another reaction site or well completely overlaps the other reaction site or well, and vice versa, or at least a portion of the reaction site can overlap at least a portion of an adjacent reaction site or well. "Chemically, biologically, or biochemically connectable" means that the reaction site is in fluid communication with another reaction site or well (i.e., fluid is free to flow from one to the other); or is fluidly connectable to the other site or well (e.g., the two are separated from each other by a wall or other component that can be punctured or ruptured, or a valve in a conduit connecting the two can be opened); or the reaction site and other site or well are arranged such that at least some chemical, biological, or biochemical species can migrate from one to the other, e.g., across a semipermeable membrane. As examples, a chip may have six reaction sites that are constructed and arranged to be aligned with the six wells of a 6-well microplate when the chip is stably connected with the microplate (e.g., positioned on top of the microplate), a chip having 96 reaction sites may be constructed and arranged such that the 96 wells are constructed and arranged to be aligned with the 96 wells of a 96-well microplate when the chip is stably connected with the microplate, etc. Of course, in some cases, the chip may be constructed and arranged such that a single reaction site of the chip is aligned with more than one microplate well and/or more than one other reaction site, and/or such that more than one microplate well and/or more than one other reaction site is aligned with a single reaction site of the chip.

Chips of the invention also may be constructed and arranged such that at least one reaction site and/or reactor of the chip is in fluid communication with, and/or chemically, biologically, or biochemically connectable to an apparatus constructed and arranged to address at least one well of a microplate, for example, an apparatus that can add species to and/or remove species from wells of microplates, and/or can test species within wells of a microplate. In this arrangement, the apparatus may add and/or remove species to/from a reaction site of a chip, and/or test species at reaction sites. In this embodiment, the reaction sites typically are arranged in alignment with wells of the microplate.

Chips of the invention can be substantially liquid-tight in one set of embodiments. As used herein, a "substantially liquid-tight chip" or a "substantially liquid-tight reactor" is a chip or reactor, respectively, that is constructed and arranged, such that, when the chip or reactor is filled with a liquid such as water, the liquid is able to enter or leave the chip or reactor solely through defined inlets and/or outlets of the chip or reactor, regardless of the orientation of the chip or reactor, when the chip is assembled for use. In this set of embodiments, the chip is constructed and arranged such that when the chip or reactor is filled with water and the inlets and/or outlets sealed, the chip or reactor has an evaporation rate of less than about 100 microliters per day, less than about 50 microliters per day, or less than about 20 microliters per day. In certain cases, a chip or reactor will exhibit an unmeasurable, non-zero amount of evaporation of water per day. The substantially liquid-tight chip or reactor can have a zero evaporation rate of water in other cases.

Chips of the invention can be fabricated using any suitable manufacturing technique for producing a chip having one or more reactors, each having one or multiple reaction sites, and the chip can be constructed out of any material or combination of materials able to support a fluidic network necessary to supply and define at least one reaction site. For example, the chip may be fabricated by etching silicon or other substrates, for example, via standard lithographic techniques. The chip may also be fabricated using microassembly or micromachining methods, for example, stereolithography, laser chemical three-dimensional writing methods, modular assembly methods, replica molding techniques, injection molding techniques, milling techniques, and the like as are known by those of ordinary skill in the art. The chip may also be fabricated by patterning multiple layers on a substrate, for example, as further described below, or by using various known rapid prototyping or masking techniques. Examples of materials that can be used to form chips include polymers, glasses, metals, ceramics, inorganic materials, and/or a combination of these. In some cases, the chip may be formed out of a material that can be etched to produce a reactor, reaction site and/or channel. For example, the chip may comprise an inorganic material such as a semiconductor, fused silica, quartz, or a metal. The semiconductor material may be, for example, but not limited to, silicon, silicon nitride, gallium arsenide, indium arsenide, gallium phosphide, indium phosphide, gallium nitride, indium nitride, other Group III/V compounds, Group II/VI compounds, Group III/V compounds, Group IV compounds, and the like, for example, compounds having three or more elements. The semiconductor material may also be formed out of combination of these and/or other semiconductor materials known in the art. In some cases, the semiconductor material may be etched, for example, via known processes such as lithography. In certain embodiments, the semiconductor material may have the from of a wafer, for example, as is commonly produced by the semiconductor industry.

In some embodiments, a chip of the invention may be formed from or include a polymer, such as, but not limited to, polyacrylate, polymethacrylate, polycarbonate, polystyrene, polyethylene, polypropylene, polyvinylchloride, polytetrafluoroethylene, a fluorinated polymer, a silicone such as polydimethylsiloxane, polyvinylidene chloride, bis-benzocyclobutene ("BCB"), a polyimide, a fluorinated derivative of a polyimide, or the like. Combinations, copolymers, or blends involving polymers including those described above are also envisioned. The chip may also be formed from composite materials, for example, a composite of a polymer and a semiconductor material.

In some embodiments, the chip, or at least a portion thereof, is rigid, such that the chip is sufficiently sturdy in order to be handled by commercially-available microplate-handling equipment, and/or such that the chip does not become deformed after routine use. Those of ordinary skill in the art are able to select materials or a combination of materials for chip construction that meet this specification, while meeting other specifications for use for which a particular chip is intended.

In certain embodiments, the chip may include a sterilizable material. For example, the chip may be sterilizable in some fashion to kill or otherwise deactivate biological cells (e.g., bacteria), viruses, etc. therein, before the chip is used or re-used. For instance, the chip may be sterilized with chemicals, radiated (for example, with ultraviolet light and/or ionizing radiation), heat-treated, or the like. Appropriate sterilization techniques and protocols are known to those of ordinary skill in the art. For example, in one embodiment, the chip is autoclavable, i.e., the chip is constructed and arranged out of materials able to withstand commonly-used autoclaving conditions (e.g., exposure to temperatures greater than about 100 °C or about 120 °C, often at elevated pressures, such as pressures of at least one atmosphere), such that the chip, after sterilization, does not substantially deform or otherwise become unusable. Another example of a sterilization technique is exposure to ozone. In another embodiment, the chip is able to withstand ionizing radiation, for example, short wavelength, high-intensity radiation, such as gamma rays, electron-beams, or X-rays. In some cases, ionizing radiation may be produced from a nuclear reaction, e.g., from the decay of ⁶⁰Co or ¹³¹Cs.

In one set of embodiments, at least a portion of the chip may be fabricated without the use of adhesive materials. For example, at least two components of a chip (e.g., two layers of the chip if the chip is a multi-layered structure, a layer or substrate of the chip and a membrane, two membranes, an article of the chip and a component of a microfluidic system, etc.) may be fastened together without the use of an adhesive material. For example, the components may be connected by using methods such as heat sealing, sonic welding, via application of a pressure-sensitive material, and the like. In one embodiment, the components may be held in place mechanically. For example, screws, posts, cantilevers, etc. may be used to mechanically hold the chip (or a portion thereof) together. In other embodiments, the two components of the chip may be joined together using techniques such as, but not limited to, heat-sealing methods (e.g., or more components of the chip may be heated to a temperature greater than the glass transition temperature or the melting temperature of the component before being joined to other components), or sonic welding techniques (e.g., vibration energy such as sound energy may be applied to one or more components of the chip, allowing the components to at least partially liquefy or soften).

In another set of embodiments, two or more components of the chip may be joined using an adhesive material. As used herein, an "adhesive material" is given its ordinary meaning as used in the art, i.e., an auxiliary material able to fasten or join two other materials together. Non-limiting examples of adhesive materials suitable for use with the invention include silicone adhesives such as pressure-sensitive silicone adhesives, neoprene-based adhesives, and latex-based adhesives. The adhesive may be applied to one or more components of the chip using any suitable method, for example, by applying the adhesive to a component of the chip as a liquid or as a semi-solid material such as a viscoelastic solid, applying the adhesive on a component using transfer tape, applying a pressure-sensitive adhesive, etc. In another embodiment, the adhesive may be applied to at least a component of the chip using a solvent-bonding system.

In some embodiments of the invention, the chip may be constructed and arranged such that one or more reaction sites can be defined, at least in part, by two or more components fastened together as previously described (i.e., with or without an adhesive). In some cases, a reaction site may be free of any adhesive material adjacent to or otherwise in contact with one or more surfaces defining the reaction site, and this can be advantageous when an adhesive might otherwise leach into fluid at the reaction site. Of course, an adhesive may be used elsewhere in the chip, for example, in other reaction sites. Similarly, in certain cases, a reaction site may be constructed using adhesive materials, such that at least a portion of the adhesive material used to construct the reaction site remains within the chip such that it is adjacent to or otherwise remains in contact with one or more surfaces defining the reaction site. Of course, other components of the chip may be constructed without the use of adhesive materials, as previously discussed.

As used herein, a "light-interacting component" is a component that interacts with light in some fashion related to chip and/or reactor function, for example, by producing light, reacting to light, causing a change in a property of light, directing light, altering light, etc., in a manner that affects a sample within a chip or reactor and/or determines something about the sample (the sample's presence, a characteristic of the sample, etc.). In one embodiment, the component produces light, such as in a light-emitting diode ("LED") or a laser. In another embodiment, the light-interacting component is a component that is sensitive to light or responds to light, such as a photodetector or a photovoltaic cell. In yet another embodiment, the light-interacting component manipulates or alters light in some fashion, for example, by focusing or collimating light, or causing light to diverge, such as in a lens, or spectrally dispersing light, such as in a diffraction grating or a prism. In another embodiment, the light-interacting component transmits or redirects the direction of light in some fashion, such as along a bent path or around a corner, for example, as in a waveguide or mirror. In yet another embodiment, the light-interacting component alters a property of light incident on the component, such as the degree of polarization or the frequency, for example, as in a polarizer or an interferometer. Other devices, or combinations of devices, are also possible. In general, the term "light-interacting component" does not encompass components or devices that passively transmit light without significant modification, alteration, or redirection, such as air, or a plane of glass or plastic (e.g., a "window"). The term "light-interacting component" also does not generally encompass components that passively absorb essentially all incident light without a response, such as would be found in an opaque material.

As used herein, the term "light" generally refers to any electromagnetic radiation. In some embodiments, the electromagnetic radiation may have wavelengths or frequencies in the optical or visual range (e.g., having a wavelength of between about 400 nm and about 700 nm), infrared wavelengths (e.g., having a wavelength of between about 300 nm and 700 nm), ultraviolet wavelengths (e.g., having a wavelength of between about 400 nm and about 10 nm), or the like. In some cases, the light may cover a range of frequencies, for example, between about 350 nm and about 1000 nm, between about 300 nm and about 500 nm, between about 500 nm and about 1 nm, between about 400 nm and about 700 nm, between about 600 nm and about 1000 nm, or between about 500 nm and about 50 nm. In other cases, the light may be monochromatic (i.e., having a single frequency or a narrow frequency distribution), for example, a frequency that is commonly produced by commercial lasers, or a frequency that a fluorescent agent is excited at. For example, the frequency may be a frequency that is centered around 366 nm, 405 nm, 436 nm, 546 nm, 578 nm, 457 nm, 488 nm, 514 nm, 532 nm, 543 nm, 594 nm, 633 nm, 568 nm, or 647 nm. The monochromatic beam of light may have a narrow distribution of frequencies. For example, 90% or 95% of the frequencies may be within ±5 mn or ±3 nm of the average frequency. In certain cases, the light may be polarized (e.g., linearly or circularly), or more than one wavelength of light may be used, for example, serially or simultaneously. In some embodiments, a light-interacting component may alter the wavelength of light within the device.

In embodiments in which a light-interacting component is provided in conjunction with a reactor, it may be positioned anywhere on or within the reactor. For example, the light-interacting component may be placed within or adjacent to a reaction site. In some cases, the light-interacting component is integrally connected with the reaction site, for example, as a wall or a surface of the reaction site.

As another example, the light-interacting component may be positioned elsewhere in, or integrally connected to, the chip, such that at least a portion of light entering the light-interacting component is in optical communication with the reaction site. As used herein, the term "optical communication" generally refers to any pathway that provides for the transport of electromagnetic radiation, such as visible light. Optical communication includes direct, "line-of-sight" communication. Optical communication may also be facilitated, for example, by the use of optical devices such as lenses, filters, optical fiber, waveguides, diffraction gratings, mirrors, beamsplitters, prisms, and the like. In some embodiments, the light-interacting component may direct light to or from more than one reaction site, or the light-interacting component may direct light from more than one light source to a reaction site. In certain embodiments, more than one light-interacting component may be present.

In some embodiments of the invention, the light-interacting component may include a waveguide. As used herein, the term "waveguide" is given its ordinary meaning in the art and is understood to include optical fibers. A waveguide can, in some cases, be anything that is able to receive light and guide or transmit at least a portion of that light to a destination that is not within "line-of sight" communication (although a waveguide can transmit light to a region that would be within line-of-sight, of course). In some cases, the waveguide is able to transport light around bends, corners, and similar obstacles without substantial losses.

In some embodiments, a waveguide may include a "core" region of material embedded or surrounded, at least in part, by a second "cladding" material, which may have a lower refractive index. The core may have any shape, for example, a slab, a strip, or a cylinder of material.

The waveguide, or at least a portion of the waveguide, may be fashioned out of any material able to transmit or light to or from the reaction site. The waveguide may be substantially transparent, or translucent in some cases. In some embodiments, the waveguide may be formed out of a silicon-based material, for example, glass, ion-implanted glass, quartz, silicon, silicon oxide, silicon nitride, silicon carbide, polysilicon, coated glass, conductive glass, indium-tin-oxide glass and the like. In other embodiments, the waveguide may comprise other transparent or translucent organic or inorganic materials. For example, in certain embodiments, the waveguide may comprise a polymer including, but not limited to, polyacrylate, polymethacrylate, polycarbonate, polystyrene, polypropylene, polyethylene, polyimide, polyvinylidene fluoride, an ion-exchanged polymer, and fluorinated derivatives of the above. Combinations, blends, or copolymers are also possible.

In one embodiment, the waveguide or a portion thereof may be surrounded by or coated with a highly reflective material, for example, silver or aluminum. In another embodiment, the waveguide may be fashioned such that it comprises a central material (e.g., a core) having a first index of refraction, and a surrounding material (e.g., a cladding) having a second index of refraction. The cladding may have an index of refraction that is less than the index of refraction of the central material. In yet another embodiment, the index of refraction of the core or the cladding may vary over the cross section. As one example, the core may be a graded index optical fiber, where the index of refraction is generally highest near the center of the core.

Under these conditions, a substantial portion of the light traveling through the central material may be internally reflected ("total internal reflection") as a result of this refractive index difference. Electromagnetic radiation entering one end of the waveguide may be confined to the central region due to the phenomenon of total internal reflection at the core-cladding boundary. The light may be transported through the core, without significant absorption by the cladding material or other surrounding materials, until it reaches the end of the waveguide, or a predetermined region of the waveguide that light is allowed to exit from. Light traveling through the central material may be directed around corners and other obstacles without a significant loss of intensity, for example, with an attenuation coefficient of less than about 10 db/cm or 20 db/cm. In another embodiment, the waveguide may have more than one central material or more than one surrounding material.

As one example of a waveguide, both the central and surrounding materials forming the waveguide may each be a glass. As another example, a waveguide may be formed out of a polymer and a silicon-based material, such that the material with the lower index of refraction surrounds the material with the higher index of refraction. As yet another example, the waveguide may be constructed out of a single material surrounded by, for example, air or a portion of the chip having a higher index of refraction than the waveguide, thus resulting in a condition where total internal reflection may occur at the waveguide/air or waveguide/chip interface.

The waveguide may be constructed by any suitable technique known to those of ordinary skill in the art, for example, by milling, grinding, or machining (e.g., by cutting or etching a channel into a chip substrate, then depositing material into the channel, optionally using a sealant). The waveguide may also be formed, for example, by depositing layers of materials during the chip fabrication process. The deposited material, in some cases, can have a higher index of refraction than the surrounding reactor substrate, thus forming a general core-cladding structure, as previously described. The waveguide may also be constructed by laser etching of materials forming the chip, such as glass or plastic, in such a way as to manipulate/alter the refractive index, relative to the surrounding material. In some cases, the refractive index of the etched/non-etched portion may be controlled so as to create a core-cladding structure.

In some embodiments, the light-interacting component may be, or include, a source of light. The light source may be any light source in optical communication with the reaction site. For example, the light source may be external or ambient light, a coherent or monochromatic beam of light such as created in an LED, or a laser such as a semiconductor laser or a quantum well laser. The light source may be integrally connected with a portion of the chip, for example, in a laser diode fabricated as part of the chip, or the light source may be separate from the chip and not integrally connected with it, but still positioned so as to allow optical communication with the reaction site. The light source may produce a single wavelength or a substantially monochromatic wavelength, or a wide range of wavelengths, as previously described. The source of light, in certain embodiments, may also be generated in a chemical reaction or a biological process, such as a chemical reaction that produces photons, for example, a reaction involving GFP ("green fluorescence protein") or luciferase, or through fluorescence or phosphorescence. For example, incident electrons, electrical current, friction, heat, chemical or biological reactions may be applied to generate light, for example, within a sample located within a reaction site, or from a reaction center located within the chip in optical communication with the reaction site.

In some embodiments, the light-interacting component may include a filter, for example, a low pass filter, a high pass filter, a notch filter, a spatial filter, a wavelength-selecting filter, or the like. The filter may be able to, for example, substantially reduce or eliminate a portion of the incident light. For example, the filter may eliminate or substantially reduce light having a wavelength below about 350 nm or greater than about 1000 nm. In another embodiment, the filter may be able to reduce noise within the incident light, or increase the signal-to-noise ratio of the incident light. In still another embodiment, the filter may be able to polarize the incident light, for example, linearly or circularly.

In some embodiments, the light-interacting component may include an optical element in optical communication with the reaction site. As used herein, an "optical element" refers to any element or device able to alter the pathway of light entering or exiting the optical element, for example, by focusing or collimating the light, or causing the light to diverge. For example, the optical element may focus the incident light to a single point or a small region, or the optical element may collimate or redirect divergent beams of light to form a parallel or converging beams of light. The term "focus" generally refers to the ability to cause rays of light to converge to a point or a small region. The term "collimate" generally refers to the ability to increase the convergence of rays of light, not necessarily to a point or a small region, for example, such that the beam focuses at an infinite distance. As one example, diverging beams of light may be collimated into parallel beams of light. In certain embodiments, the optical element may disperse or cause light to diverge, for example, as in a diverging lens. In other embodiments, the optical element may be, for example, a beamsplitter, an optical coating (e.g., a dichroic, an antireflective, or a reflective coating), an optical grating, a diffraction grating, or the like.

In one set of embodiments, the optical element may be a lens. The lens may be any lens, such as a converging or a diverging lens. The lens may be, for example, a meniscus, a plano-convex lens, a plano-concave lens, a double convex lens, a double concave lens, a Fresnel lens, a spherical lens, an aspheric lens, a binary lens, or the like. The optical element may also be a mirror, such as a planar mirror, a curved mirror, a parabolic mirror, or the like. In other embodiments, the optical element may cause light to disperse, for example, as in a diffraction grating or a prism.

In certain embodiments, a material having an index of refraction may be used as the optical element. The optical element may affect the incident light due to differences in the indexes of refraction. For example, in embodiments in which light reaches the optical element through a waveguide, the optical element may be a material having a different index of refraction than the waveguide. In some cases, the index of refraction of the optical element will be about the same as or more than the index of refraction of the waveguide, allowing at least a portion of the light traveling along the waveguide to enter or pass through the optical element.

In certain embodiments, a material having a graded index of refraction may be used as an optical element. This material can be referred to as a "graded index material" or a "GRIN" material. The GRIN material may minimize the amount of divergence inherent in light reaching the GRIN material. For example, a material of uniform thickness can be made to act as a lens by varying its refractive index along a cross section of the element. In another embodiment, the GRIN material may redirect divergent rays of light into a parallel arrangement. In another embodiment, the GRIN material does not necessarily have a uniform thickness, and a combination of the graded index of refraction of the material and the shape of the material may be used to focus or collimate the light

The light-interacting component, in some embodiments, may include a component that is able to convert light to electricity, such as a photosensor or photodetector, a photomultiplier, a photocell, a photodiode such as an avalanche photodiode, a photodiode array, a CCD chip ("charge-coupled device") or the like. The component may be used, in some cases, to determine the state or condition of a substance within a reaction site, for example, through emission (including fluorescence or phosphorescence), absorbance, scattering, optical density, polarization measurements, or other measurements, including using the human eye.

In other cases, the component may be used for imaging purposes, for example, to image a portion of a cell or other material located at or near the reaction site, or to determine whether a cell has adhered to a surface.

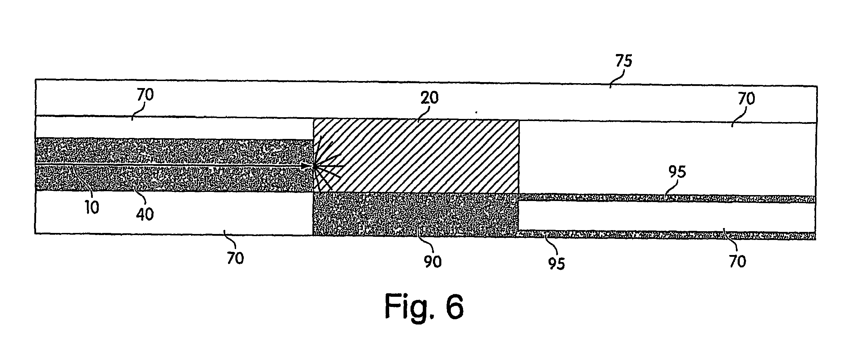

In some cases, the component may be used to produce electricity. In one embodiment, a photocell may be integrally fabricated within the chip using one or more layers comprising semiconductor materials, as shown in Fig. 6.

In some embodiments, light is directed to the reaction site, for example, to activate or inhibit a chemical reaction. For example, a reaction may require the use of light for activation, or a light-sensitive enzyme may be inhibited by applying light to the enzyme. In certain embodiments, light directed to the reaction site may be used as a probe or a signal source. The light may be delivered in a controlled manner to the reaction site in certain embodiments, for example, so that the light reaching the reaction site has a specific wavelength, polarization, or intensity.

In some embodiments, a portion of the light arising from the reaction site is detected and analyzed. The light arising from the reaction site may be reflected or refracted light, for example, light directed to the reaction as previously described, or the light may be produced through physical means, for example, through fluorescence or phosphorescence. In certain embodiments, the light may be generated within the reaction site, as previously described. Light from the reaction site may be analyzed using any suitable analytical technique, for example, infrared spectroscopy, FTIR ("Fourier Transform Infrared Spectroscopy"), Raman spectroscopy, absorption spectroscopy, fluorescence spectroscopy, optical density, circular dichroism, light scattering, polarimetry, refractometry, turbidity measurements, quasielectric light scattering, or any other suitable techniques. In another embodiment, imaging of the reaction site may be performed, for example using optical imaging, or infrared imaging.

Fig. 1 illustrates one embodiment of the invention. Entering light 10 enters reaction site 20, where it interacts with a sample 25, producing exiting light 15. The interaction may include, for example, reflection, scattering, refraction, polarization, absorption and reemission (e.g., at a different frequency), or the like. Exiting light 15 then interacts with detector 30. Detector 30 may be positioned anywhere such that it remains in optical communication with reaction site 20. As one example, in Fig. 2, detector 30 is positioned such that it can measure the "scatter" (e.g., at any angle other than 180°) of a portion of light 15 exiting reaction site 20. Detector 30 may be integrally connected to the reaction site 20 in some embodiments; in other embodiments, detector 30 may be separate from reaction site 20.

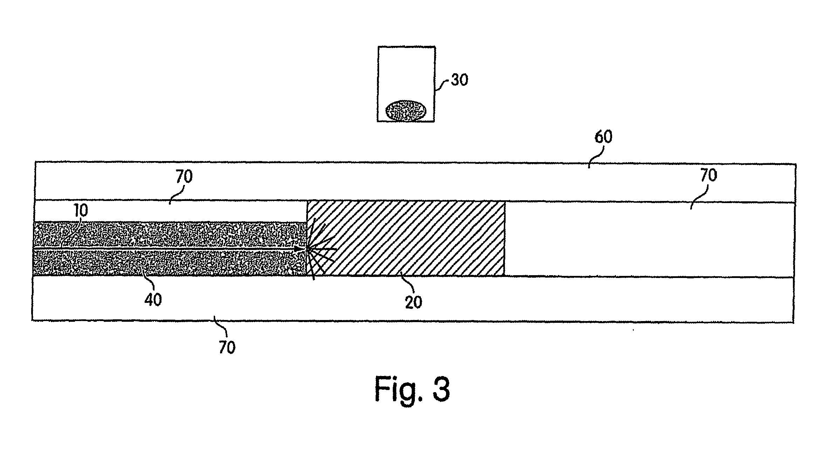

Fig. 3 illustrates another embodiment of the invention, as used within a chip. In Fig. 3, incident light 10 enters a reaction site 20 through a waveguide 40. The chip also has a covering 60, which may be, for example, transparent or translucent. Covering 60 allows detector 30 to be in optical communication with reaction site 20. Detector 30 may detect light (or the absence thereof) arising from reaction site 20, which, in this embodiment, results from incident light 10. Other components of the chip, which may, for example, define reaction site 20, are also illustrated in Fig. 3. For example, component 70 may comprise silicon, glass, a plastic, or other substances as previously described.

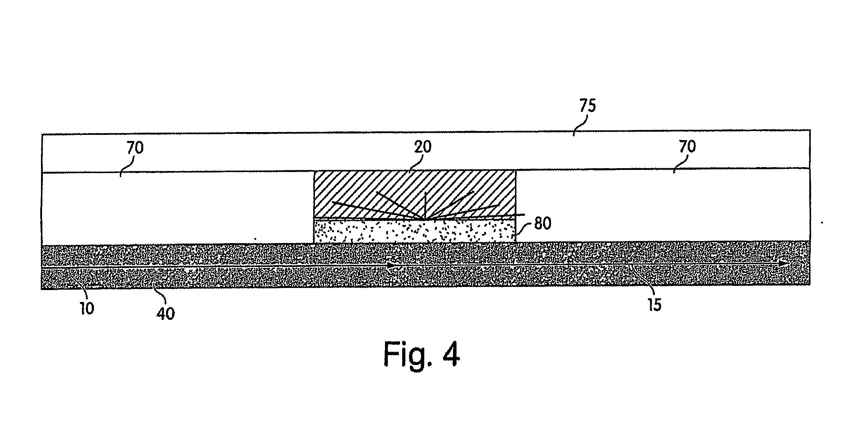

A different configuration is shown in Fig. 4. In Fig. 4, entering light 10 is in optical communication with reaction site 20 (defined by substrates 70 and 75) through optical element 80, which may be, for example, a diverging lens or a material of a refractive index which may be of a higher value than the refractive index of waveguide 40. At least a portion of incident light 10 traveling through waveguide 40 reaches focusing unit 80, and from there is transmitted through focusing unit 80 into reaction site 20. The remaining light 15 then continues through waveguide 40, for example, proceeding to another reaction site or to a detection unit. Light entering reaction site 20 may then be detected by a detection unit (not shown), or the light may interact with a sample (not shown) contained within reaction site 20. In some cases, light entering reaction site 20 may be redirected back towards optical element 80 or waveguide 40. Thus, the sample is able to interact with, or be in optical communication with, entering light 10 to produce exiting light 15 in some cases. In other embodiments, substrate 75 may be substantially transparent or translucent, for example, being constructed out of glass or a clear plastic, such that the contents of reaction site 20 may be observed or detected, either directly, by means of a detector (not shown), or by using additional instruments such as a microscope.

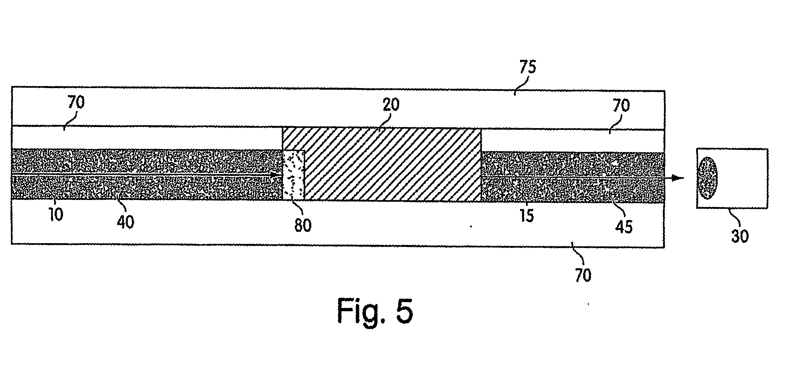

Another embodiment of the invention is shown in Fig. 5. In this embodiment, light 10 traveling through waveguide 40 is collimated by optical element 80, which may be, for example, a graded index lens or a collimating mirror. Light then enters reaction site 20. A portion of the entering light reaches waveguide 45, where it is transmitted away from reaction site 20 as light 15. Light 15 then reaches photodetector 30, which may or may not be integrally connected with substrates 70 and 75. In some embodiments, a second detector, for example a microscope or an additional photodetector, may be used in conjunction with a transparent or translucent substrate 75 for additional analysis of reaction site 20, for example, as previously discussed in reference to Fig. 3.

In Fig. 6, detector 90 has been formed as an integral part of reaction site 20. In this embodiment, detector 90 forms at least one surface defining reaction site 20. Detector 90 may be constructed as an integral part of reaction site 20 using any suitable technique, for example, by layering down multiple semiconductor materials within the chip. In this embodiment, light 10, entering through waveguide 40, enters reaction site 20. At least a portion of the entering light is then transmitted to detector 90, for example, through refraction, reflection, or scattering. Detector 90, in this embodiment, is in electronic communication with an external device through the use of wires 95. Leads are formed as part of substrate 70 in this particular embodiment. Substrate 75, which may be the same or different than substrate 70, may also be transparent or substantially transparent in certain cases, for example, to facilitate additional analysis, as previously discussed in reference to Fig. 3.

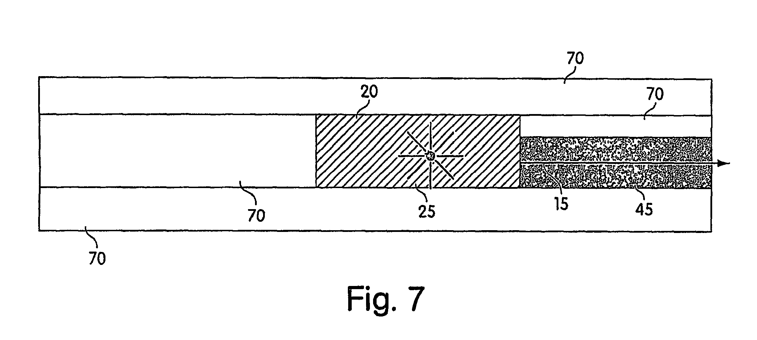

Fig. 7 illustrates another embodiment of the invention, in which reaction site 20 contains sample 25 that is able to produce light, for example, through the application of heat, sound, pressure, electricity, or through the use of a chemical or biological reaction. For example, sample 25 may represent a photoluminescent reaction or a biological cell able to produce light. In Fig. 7, at least a portion of the light arising from sample 25 is collected by waveguide 45 as light 15, and is transmitted from reaction site 20, for example, to a detector or to another reaction site.

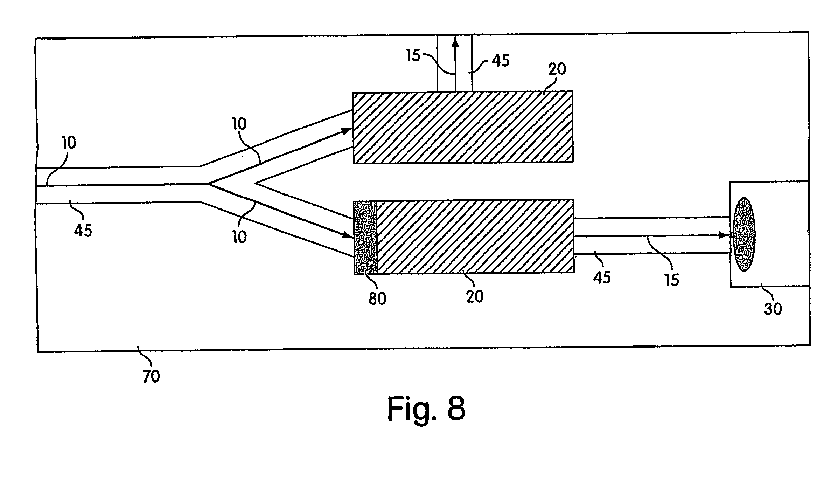

As previously described, the chip may include multiple reaction sites, multiple light-interacting components, or a combination of these. In some embodiments, the chip may include several reaction sites, each in optical communication with, for example, a single waveguide, a single detector, or a single light source. In other embodiments, a reaction site may be in optical communication with more than one light-interacting component, for example, two light sources, a light source and an optical element, or the like. For example, Fig. 8 illustrates an embodiment of the invention having more than one reaction site. In this embodiment, light 10 in waveguide 40 is directed at two different reaction sites 20. Light from each of reaction sites 20 is then directed to waveguides 45. In this embodiment of the invention, one reaction site has optical element 80. Light from one chamber reaches photodetector 30, integrally connected with substrate 70 in this illustration, while light from the other chamber is directed externally from substrate 70.

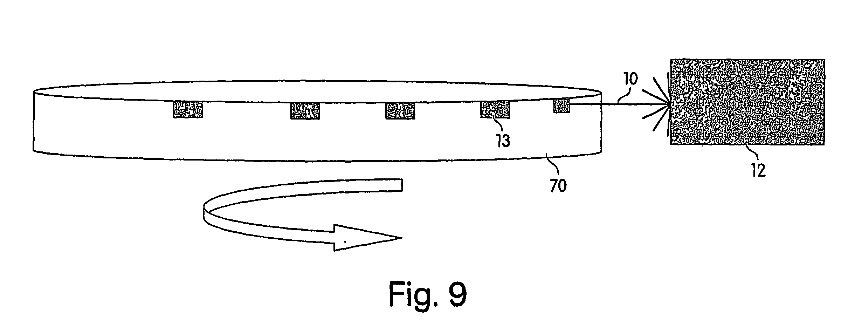

Fig. 9 shows another embodiment of the invention, in which substrate 70 is able to rotate. Light source 12, producing entering light 10, reaches a plurality of entities 13 contained within substrate 70. Entities 13 may represent, for example, reaction sites, waveguides that lead to reaction sites, or a combination of these or other elements. As substrate 70 is rotated, light 10 from light source 12 either enters entity 13, or is deflected or absorbed by the substrate 70. As one example, the substrate may be coated with a reflective surface to deflect light 10, or an absorptive surface to absorb light 10. Thus, in this embodiment, one light source 12 may interact with more than one reaction site.

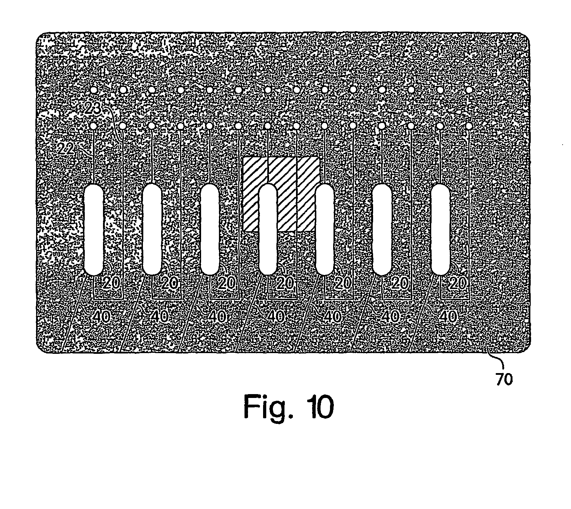

Fig. 10 shows another embodiment of the invention. This embodiment consists of substrate 70, which defines a plurality of reaction sites 20. An additional layer (not shown) comprises the bottom of the reaction site. A second additional layer (not shown) is transparent to visible light and comprises the top of the reaction site. Each reaction site is in fluid communication with inlet 22 and outlet 23. The inlets and outlets may be used, for example, to introduce or remove various reactants, fluids, carriers, cells, and the like. Waveguides 40 may be fabricated in substrate 70 so as to be in optical communication with reaction sites 20 and the edge of substrate 70. In one embodiment, a light source (not shown) can be directed into the waveguides from the edge of substrate 70, such that a substantial portion of the directed light is transported to reaction sites 20. Light scattered by the contents of reaction site 20 may then be detected by a photodetector (not shown), which may be integral connected to substrate 70, or located above the transparent top.

The chip can include a variety of components for sensing, actuation, or other activity. For example, the chip may include components such as a membrane, a lens, a light source, a waveguide, a circuit such as an integrated circuit, a reservoir (e.g., for a solution), a micromechanical or a MEMS ("microelectromechanical system") component, a control system, or the like, for example, as further described below. In some embodiments, at least one, two, three or more components are integrally connected to the chip. In certain embodiments, all of the components are integrally connected to the chip.

Other examples of components suitable for use with the invention include pylon-like obstructions placed in the flow path of a stream to enhance mixing within the chip, reactor and/or reaction site, or heating, separation, and/or dispersion units within the chip, reactor and/or reaction site. For example, if a heating unit is present, the heating unit may be a miniaturized, traditional heat exchanger.

In one set of embodiments, the present invention includes a membrane, such as a membrane that may be substantially transparent. If a membrane is present, it may be positioned anywhere in a reactor within a chip. In one embodiment, the membrane is positioned such that it defines the surface of one or more reaction sites. In another embodiment, the membrane can be positioned such that it is in fluidic communication with one or more reaction sites of the chip. In some cases, the membrane may be positioned such that a pathway fluidly connecting a first reaction site with a second reaction site crosses the membrane. As used herein, a "substantially transparent" membrane is a membrane that allows electromagnetic radiation to be transmitted through the membrane without significant scattering, such that the intensity of electromagnetic radiation transmitted through the membrane is sufficient to allow the radiation to interact with a substance on the other side of the membrane, such as a chemical, biochemical, or biological reaction, or a cell. In some cases, the membrane is substantially transparent to incident electromagnetic radiation ranging between the infrared and ultraviolet ranges (including visible light) and, in particular, between wavelengths of about 400 - 410 nm and about 1,000 nm. The substantially transparent membrane may be able to transmit electromagnetic radiation in some cases such that a majority of the radiation incident on the membrane passes through the membrane unaltered, and in some embodiments, at least about 50%, in other embodiments at least about 75%, in other embodiments at least about 80%, in still other embodiments at least about 90%, in still other embodiments at least about 95%, in still other embodiments at least about 97%, and in still other embodiments at least about 99% of the incident radiation is able to pass through the membrane unaltered. In certain cases, the membrane is at least partially transparent to electromagnetic radiation within the above-mentioned wavelength range to the extent necessary to promote and/or monitor a physical, chemical, biochemical, and/or biological reaction occurring within a reaction site, for example as previously described. In other embodiments, the membrane may be transparent to electromagnetic radiation within the above-mentioned wavelength range to the extent necessary to monitor, observe, stimulate and/or control a cell within the reaction site.

In yet another set of embodiments, the membrane may be a porous membrane having, for example, a number-average pore size of greater than about 0.03 micrometers and less than about 2 micrometers. In other embodiments, the pore size of the membrane may be less than about 1.5 micrometers, less than about 1.0 micrometers, less than about 0.75 micrometers, less than about 0.5 micrometers, less than about 0.3 micrometers, less than about 0.1 micrometers, less than about 0.07 micrometers, and in other embodiments, less than about 0.05 micrometers. In certain cases, the pores are also greater than 0.03 micrometers or greater than 0.08 micrometers.

In certain embodiments, the membrane may be formed out of a substance that has a number-average pore size, as previously described, that is also substantially transparent, as previously described. For example, the porous substantially transparent membrane may include polymers such as polyethylene terephthalate (PET), polysulfone, polycarbonate, acrylics such as polymethyl methacrylate, polyethylene, polypropylene, and the like. In one embodiment, the substantially transparent membrane is a polyethylene terephthalate membrane having a pore size of 2 micrometers or less.

In one set of embodiments, a chip of the invention may include a structure adapted to facilitate the reactions or interactions that are intended to take place therein (e.g., within a reaction site). For example, where a chip is intended to function as one or more bioreactors for cell culturing, the chip may include structure(s) able to improve or promote cell growth. For instance, in some cases, a surface of a reaction site may be a surface able to promote cell binding or adhesion, or the reactor and/or reaction site within the chip may include a structure that includes a cell adhesion layer, which may include any of a wide variety of hydrophilic, cytophilic, and/or biophilic materials. As examples, the surface may be ionized, or coated and/or micropatterned with any of a wide variety of hydrophilic, cytophilic, and/or biophilic materials, for example, materials having exposed carboxylic acid, alcohol, and/or amino groups. Examples of materials that may be suitable for a cell adhesion layer include, but are not limited to, polyfluoroorganic materials, polyester, PDMS, polycarbonate, polystyrene, and aluminum oxide. As another example, the structure may include a layer coated with a material that promotes cell adhesion, for example, an RGD peptide sequence, or the structure may be treated in such a way that it is able to promote cell adhesion, for example, the surface may be treated such that the surface becomes relatively more hydrophilic, cytophilic, and/or biophilic. In some embodiments, it may be desired to modify the surface of a cell adhesion layer, for instance with materials that promote cell adhesion, for example, by attachment, binding, soaking or other treatments. Example materials that promote cell adhesion include, but are not limited to, fibronectin, laminin, albumin or collagen. In other embodiments, for example, where certain types of bacteria or anchorage-independent cells are used, the surface may be formed out of a hydrophobic, cytophobic, and/or biophobic material, or the surface may be treated in some fashion to make it more hydrophobic, cytophobic, and/or biophobic, for example, by using aliphatic hydrocarbons and/or fluorocarbons.

In some embodiments of the invention, a reactor and/or a reaction site within a chip may be constructed and arranged to maintain an environment that promotes the growth of living cells. In embodiments where one or more cells are used in the reaction site, the cells may be any cell or cell type. For example, the cell may be a bacterium or other single-cell organism, a plant cell, or an animal cell. If the cell is a single-cell organism, then the cell may be, for example, a protozoan, a trypanosome, an amoeba, a yeast cell, algae, etc. If the cell is an animal cell, the cell may be, for example, an invertebrate cell (e.g., a cell from a fruit fly), a fish cell (e.g., a zebrafish cell), an amphibian cell (e.g., a frog cell), a reptile cell, a bird cell, or a mammalian cell such as a primate cell, a bovine cell, a horse cell, a porcine cell, a goat cell, a dog cell, a cat cell, or a cell from a rodent such as a rat or a mouse. If the cell is from a multicellular organism, the cell may be from any part of the organism. For instance, if the cell is from an animal, the cell may be a cardiac cell, a fibroblast, a keratinocyte, a heptaocyte, a chondracyte, a neural cell, a osteocyte, a muscle cell, a blood cell, an endothelial cell, an immune cell (e.g., a T-cell, a B-cell, a macrophage, a neutrophil, a basophil, a mast cell, an eosinophil), a stem cell, etc. In some cases, the cell may be a genetically engineered cell. In certain embodiments, the cell may be a Chinese hamster ovarian ("CHO") cell or a 3T3 cell. In some embodiments, more than one cell type may be used simultaneously, for example, fibroblasts and hepatocytes. In certain embodiments, cell monolayers, tissue cultures or cellular constructs (e.g., cells located on a non-living scaffold), and the like may also be used in the reaction site. The precise environmental conditions necessary in the reaction site for a specific cell type or types may be determined by those of ordinary skill in the art.

In some instances, the cells may produce chemical or biological compounds of therapeutic and/or diagnostic interest. For example, the cells may be able to produce products such as monoclonal antibodies, proteins such as recombinant proteins, amino acids, hormones, vitamins, drug or pharmaceuticals, other therapeutic molecules, artificial chemicals, polymers, tracers such as GFP ("green fluorescent protein") or luciferase, etc. In one set of embodiments, the cells may be used for drug discovery and/or drug developmental purposes. For instance, the cells may be exposed to an agent suspected of interacting with the cells. Non-limiting examples of such agents include a carcinogenic or mutagenic compound, a synthetic compound, a hormone or hormone analog, a vitamin, a tracer, a drug or a pharmaceutical, a virus, a prion, a bacteria, etc. For example, in one embodiment, the invention may be used in automating cell culture to enable high-throughput processing of monoclonal antibodies and/or other compounds of interest.

In certain cases, a reactor and/or a reaction site within a chip may be constructed and arranged to prevent, facilitate, and/or determine a chemical or a biochemical reaction with the living cells within the reaction site (for example, to determine the effect, if any, of an agent such as a drug, a hormone, a vitamin, an antibiotic, an enzyme, an antibody, a protein, a carbohydrate, etc. on a living cell). For example, one or more agents suspected of being able to interact with a cell may be added to a reactor and/or a reaction site containing the cell, and the response of the cell to the agent(s) may be determined, using the systems and methods of the invention.

In some cases, the cells may be sensitive to light. For example, the cell may be a plant cell that responds to a light stimulus or is photosynthetic. In another embodiment, the light may be used to grow cells, such as mammalian cells sensitive to light, or plant cells. In yet another embodiment, the cell is a bacterium that is attracted to or is repelled by light. In another embodiment, the cell is an animal cell having a light receptor or other light-signaling response, for example, a rod cell or a cone cell. In yet another embodiment, the cell is a genetically engineered cell having a light receptor or another light-sensitive molecule, for example, one that decomposes or forms reactive entities upon exposure to light, or stimulates a biological process to occur. In other cases, the cell may be insensitive to light; light applied to the chip may be used for analysis of the cells, for example, detection, imaging, counting, morphological analysis, or spectroscopic analysis. In still other cases, the light may be used to kill the cells, for example, directly, or by inducing an apoptotic reaction.

In some embodiments, the chip is constructed and arranged such that cells within the chip can be maintained in a metabolically active state, for example, such that the cells are able to grow and divide. For instance, the chip may be constructed such that one or more additional surfaces can be added to the reaction site, for example, as in a series of plates, or the chip may be constructed such that the cells are able to divide while remaining attached to a substrate. In some cases, the chip may be constructed such that cells may be harvested or removed from the chip, for example, through an outlet of the chip, or by removal of a surface from the reaction site, optionally without substantially disturbing other cells present within the chip. The chip may be able to maintain the cells in a metabolically active state for any suitable length of time, for example, 1 day, 1 week, 30 days, 60 days, 90 days, 1 year, or indefinitely in some cases.

In one set of embodiments, the chip is able to control an environmental factor associated with a reaction site by transporting an agent into or proximate the reaction site. Control of the delivery of the agent to the reaction site, in certain instances, may be used to control the environmental factor. In some cases, the chip is able to control the environmental factor without directly contacting the reaction site to an external or unsterilized agent, such as a liquid. As used herein, an "environmental factor" is a detectable or measurable condition of the environment associated with the reaction site, such as pH or the concentration of a compound. The factor or condition may be one located within the reaction site, and/or at a location relative to the reaction site (e.g., upstream or downstream) such that the environment within the reaction site is known or controlled. For example, the environmental factor may be an aggregate quantity, such as molarity, osmolarity, salinity, total ion concentration, pH, or color. The concentration may also be the concentration of one or more compounds present within the reaction site, for example, an ion concentration such as sodium, potassium, calcium, iron or chloride ions; or a concentration of a biologically active compound, such as a protein, a lipid, or a carbohydrate source (e.g., a sugar) such as glucose, glutamine, pyruvate, apatite, an amino acid or an oligopeptide, a vitamin, a hormone, an enzyme, a protein, a growth factor, a serum, or the like. In some embodiments, the substance within the reaction site may include one or more metabolic indicators, for example, as would be found in media, or as produced as a waste products from cells.

Thus, in some cases, the environmental factor within the reaction site may be altered and/or controlled without directly contacting the reaction site with a liquid agent. In one embodiment, the chip may be constructed to allow an agent to permeate or diffuse into the reaction site. For instance, the reaction site may include a component such as a wall or a layer of the chip, through which an agent is able to permeate. The agent may be able to alter and/or control one or more environmental factors. For instance, the agent may be a non-pH-neutral composition or a pH-altering agent. As another example, the component may include a membrane, such as an osmotic membrane or a semipermeable membrane (e.g., with respect to the agent) that the agent is able to permeate across. In some cases, the component may be chemically or physically inert with respect to the agent. In certain instances, a flow of agent may occur on one side of the component. In some embodiments, the flow of agent on one side of the component may occur along a meandering or non-straight pathway, for example, to increase the time of contact between the agent and the component.

In some cases, the component may comprise a polymer that a molecule is able to permeate. For example, the polymer may include nylon, polyethylene, polypropylene, polycarbonate, polydimethylsiloxane, or copolymers or blends. In another set of embodiments, the component may include a polymer substantially impermeable to the agent being transported, but the component may be constructed or designed to allow transport of the agent to occur, for example, through a region that is porous or contains a number of channels. In another embodiment, the component may be impermeable to the agent being transported, but the component may be converted to a permeable form upon the addition of a permeabilizing agent. As used herein, "permeation" and "permeate" refer to any non-bulk transport process. A non-bulk transport generally is a transport process where substantial convection or bulk flow does not occur. For example, permeation of the agent may occur through passive diffusion, or through pores or other interstices; or transport may be facilitated or enhanced in some manner, for instance, through osmosis, electrodiffusion, electroosmosis, percolation, or through the use of a permeation-enhancing compound. In some embodiments, transport may be facilitated using an externally-applied field, such as an electrical, magnetic, or a centripetal field.

In some cases, the component may be designed to transport an agent across the component within a given period of time or under a certain condition. The exact desired thickness, density, porosity, tortuosity, composition, or other characteristics of the component may be determined by those of ordinary skill in the art. In certain cases, transport of the agent may be relatively rapid. For instance, the component may be constructed such that an agent is transported across in less than about 10 minutes, less than about 5 minutes, less than about 3 minutes, or less than about 1 minute.

In some embodiments, an environmental factor within the reaction site may be altered by generating one or more chemical agents within the chip, for example, from a precursor, that interact with, or alter in some way, an environmental factor associated with the reaction site. In one embodiment, the chemical agent may be generated within the reaction site. In another embodiment, the chemical agent may be generated elsewhere and transported to the reaction site. For example, the chemical agent may be produced and/or stored within a different compartment associated with or external of the chip (e.g., as in a reservoir), then transported to the reaction site, for instance, through a channel or other fluidic connection, or by allowing it to permeate or diffuse across a membrane or another component. In one embodiment, the agent may be generated in a location proximate the reaction site, e.g., such that it can be transferred to the reaction site, for example, in a few seconds. In another embodiment, the agent may be a gas transported to the reaction site, for example, through a membrane, or over a barrier that prevents liquid communication between the compartment and the reaction site. The reaction may be externally initiated in certain embodiments. For example, a light source, such as a laser, may be applied to the reactants, or heat may be used to initiate a reaction. In yet another embodiment, a fluidic connection may be created between the compartment and the reaction site, for example, reversibly. For instance, the fluidic connection may be created by opening a valve such as a mechanical valve or a micromechanical valve, etc. separating the compartment and the reaction site.

In some cases, additional compounds may be combined with the precursors to, for example, preserve the precursors against decomposition, to enhance the ability of the precursors to react (e.g., a catalyst or an enzyme), or to enhance the absorption of incident energy onto the chemical, for instance, to increase the chemical reaction rate. In one set of embodiments, a material that is absorptive of incident electromagnetic radiation is a darkened or "black" material which may be added to the precursors, for example, to enhance the absorption of light energy. Non-limiting examples of black material include quartz, black glass, silicon, black sand, carbon black, and the like. The additional compounds may be substantially unreactive, unable to form a transportable agent, or the additional compounds may not significantly interfere with the production of the agents or control of the environmental factors associated with the reaction site. The chemical agent, in certain embodiments, may be produced in a reaction that is activated at a certain temperature, such as in a thermal decomposition or degradation reaction. In some cases, the reaction may be initiated when the precursors are exposed to at least a certain temperature. The temperature necessary to activate the reaction may be produced, for example, upon the application of light energy, heat, an exothermic chemical reaction, or the like. In some instances, the generated chemical agent may be a gas, for example, O₂, CO, CO₂, NO, NO₂, ammonia, acetic acid, or the like. In some cases, the chemical reaction may produce one or more gases and/or one or more non-gaseous products. The gases may then be transported into the reaction site (for example, through a membrane or over a barrier), while non-gaseous products may be prevented from entering the reaction site.

Chips of the invention typically include or are connected to one or more fluid pathways for delivery of species or removal of species from a reaction site. In some cases, a fluidic pathway can be created *in situ* (after construction of the chip, during chip setup and/or during use of the chip) by permeabilizing or damaging a component separating the compartment from the reaction site (e.g., as in a wall or a membrane), or separates the compartment from a fluidic pathway in fluid communication with the reaction site. For example, the component may be permeabilized by heating the component to increase the permeability of the chemical agent, or by causing the component to melt or vaporize. The component, in some cases, may also be dissolved or damaged through a reaction, for example, a chemical or electrochemical reaction, to produce a fluidic connection with the reaction site. For example, the component may include a metal, such as gold, silver or copper, that can be electrolyzed upon the application of a suitable electrical current. As another example, the component may be chemically etched, for example, with a reactive species. In still other embodiments, the component may be mechanically damaged, for example, by piercing the surface with a microneedle, which may originate from within the chip, or externally. The component, may also be damaged without the use of mechanical forces or chemicals. For example, energy may be applied to the surface to damage it. In one embodiment, the component may be ablated, for example, using light. If light is used, the light may be channeled through a waveguide to the surface in some cases, or light may be applied directly to the surface. In some cases, the permeability of the component may be enhanced by one, two, or three or more orders of magnitude. In certain cases, the enhancement may be reversible, for example, by decreasing temperature, or introducing a non-permeabilizing agent. The component may include a material able to enhance the creation of the fluidic pathway in some cases. For example, the material may facilitate the absorption of light energy, or increase the chemical reaction or transport rate. For instance, in one embodiment, the surface comprises a material that is absorptive of incident electromagnetic radiation, such as quartz, black glass, silicon, black sand, carbon black, etc. As another example, the component may include a catalyst, an enzyme, or a permeation enhancer.

In some aspects of the invention, any of the above-described chips may be constructed and arranged such that the chip is able to respond to a change in an environmental condition within or associated with a reaction site, for example, by use of a control system. Detection of the environmental condition may occur, for example, by means of a sensor which may be positioned within the reaction site, or positioned proximate the reaction site, i.e., positioned such that the sensor is in communication with the reaction site in some manner (for example, fluidly, optically, thermally, pneumatically, electronically etc.) to the extent that it can sense one or more conditions that it is designed to sense within or associated with the reaction site. The sensor may be, for example, a pH sensor, an oxygen sensor, a sensor able to detect the concentration of a substance, or the like. The sensor may be embedded and integrally connected with the chip (e.g., within a component defining at least a portion of the reaction site a channel in fluidic communication with the reaction site, etc., or separate from the chip. In certain embodiments, the sensor may be a ratiometric sensor, i.e., a sensor able to determine a difference or ratio between two (or more) measurements.

Chips and reactor systems of the invention also can include a control system(s). As used herein, a "control system" is a system able to detect and/or measure one or more environmental factors within or associated with the reaction site, and cause a response or a change in the environmental conditions within or associated with the reaction site (for instance, to maintain an environmental condition at a certain value). The response produced by the control system may be based on the environmental factor in certain cases. An "active" control system, as used herein, is a system able to cause a change in an environmental factor associated with a reaction site as a direct response to a measurement of the environmental condition. The active control system may provide an agent that can be delivered, or released from the reaction, where the agent is controlled in response to a sensor able to determine a condition associated with the reaction site. A "passive" control system, as used herein, is a system able to maintain or cause a change in an environmental condition of the reaction site without requiring a measurement of an environmental factor. The passive control system may control the environmental factor within the reaction site, but not necessarily in response to a sensor or a measurement. The passive control system may allow an agent to enter or exit the reaction site without active control. For example, a passive control system may include an oxygen membrane and/or a water-permeable membrane, where the membrane can maintain the oxygen and/or the water vapor content within the reaction site, for instance, within certain predetermined limits. The control system may be able to control one or more conditions within or associated with the reaction site for any length of time, for example, 1 day, 1 week, 30 days, 60 days, 90 days, 1 year, or indefinitely in some cases.

For example, in one embodiment, the control system comprises a membrane including a humidity control material, for example, in a membrane having a permeability to oxygen high enough, and a permeability to water vapor low enough, to allow cell culturing. Non-limiting examples of such materials include poly(4-methyl pentene-1), poly(1-trimethylsilyl-1-propyne), and BIOFOIL^{®} polymer membrane, made by VivaScience (Hannover, Germany). The humidity control material may allow the passage of desired gases therethrough, such as oxygen, while inhibiting the passage of other gases, for example, water vapor. For example, the humidity control material may have a permeability to water that is less than about 0.39 mol/day/m² and, in other embodiments, less than about 0.35 mol/day/m² or less than about 0.1 mol/day/m², and an oxygen permeability of at least about 0.061 mol/day/m²/atm, and in some embodiments, at least about 0.7 mol/day/m²/atm, and in other embodiments greater than about 0.8 or 0.9 mol/day/m²/atm. In one embodiment, the present invention achieves a certain permeability by combining two or more layers or portions of material. For example, in one embodiment where the humidity control material comprises at least two layers, the layers may be formed out of the same or distinct polymers.

As used herein, a "processor" or a "microprocessor" is any component or device able to receive a signal from one or more sensors, store the signal, and/or convert the signal into one or more responses for one or more actuators, for example, by using a mathematical formula, or an electronic or computational circuit. The signal may be any suitable signal indicative of the environmental factor determined by the sensor, for example a pneumatic signal, an electronic signal, an optical signal, a mechanical signal, etc. The processor may be any device suitable for determining a response to the signal, for example, a mechanical device, or an electronic device such as a semiconductor chip. The processor may be embedded and integrally connected with the reaction site or chip or separate from the reaction site or chip, depending on the application. In one embodiment, the processor is programmed with a process control algorithm, which can, for example, take an incoming signal from a sensor and convert the signal into a suitable output for an actuator. Any suitable algorithm(s) may be used within the processor, for example, a PID control system, a feedback or feedforward system, a fuzzy logic control system, etc. The processor may be programmed or otherwise designed to control an environmental condition within the reaction site, for example, by manipulation of an actuator.

As used herein, an "actuator" is a device able to affect the environment within or proximate to one or more reaction sites, or in an inlet or outlet in fluid communication with one or more reaction sites. The actuator may be separate from, or integrally connected to the reaction site or chip. For example, in some embodiments, the actuator may include a valve or a pump able to control, alter, and/or prevent the flow of a substance or agent into or out of the reaction site, for example, a chemical solution, a buffering solution, a gas such as CO₂ or O₂, a nutrient solution, a saline solution, an acid, a base, a solution containing a carbon source, a nitrogen source, an inhibitor, a promoter, a hormone, a growth factor, an inducer, etc. The substance to be transported will depend on the specific application.

In some cases, the pump may be external of the chip. As one example, the actuator may selectively open a valve that allows CO₂ or O₂ to enter the reaction site. In other cases, the pump may be internal of the chip. For example, the pump may be a piezoelectric pump or a mechanically-activated pump (e.g., activated by pressure, electrical stimulation, etc.). In one embodiment, the pump is activated by producing a gas within the chip, which may cause fluid flow within the chip; as examples, the gas may be produced by directing light such as laser light at a reactant to start a gas-producing reaction, or the gas may be produced by applying an electric current to a reactant (for instance, an electric current may be applied to water to produce gas). As another example, the actuator may include a pumping system that can create a fluid connection with a reaction site as necessary.

In one aspect, the present invention provides any of the above-mentioned chips packaged in kits, optionally including instructions for use of the chips. That is, the kit can include a description of use of the chip, for example, for use with a microplate, or an apparatus adapted to handle microplates. As used herein, "instructions" can define a component of instruction and/or promotion, and typically involve written instructions on or associated with packaging of the invention. Instructions also can include any oral or electronic instructions provided in any manner such that a user of the chip will clearly recognize that the instructions are to be associated with the chip. Additionally, the kit may include other components depending on the specific application, for example, containers, adapters, syringes, needles, replacement parts, etc. As used herein, "promoted" includes all methods of doing business including methods of education, hospital and other clinical instruction, scientific inquiry, drug discovery or development, academic research, pharmaceutical industry activity including pharmaceutical sales, and any advertising or other promotional activity including written, oral and electronic communication of any form, associated with the invention.

The function and advantage of these and other embodiments of the present invention will be more fully understood from the examples below. The following examples are intended to illustrate the benefits of the present invention, but do not exemplify the full scope of the invention.

### Example 1

In this example, a chip, as illustrated generally in Fig. 10, was prepared in accordance with an embodiment of the invention.

A first chip layer having associated fluidic channels, ports, chambers, other reaction sites, etc. therein was injection molded or machined from a stock sheet of acrylic or polycarbonate. This first layer was attached to a machined or injection molded flat bottom plate (also acrylic or polycarbonate) by means of a pressure-sensitive silicone adhesive (Dielectric Polymers). A 0.2 micrometer pore size membrane (Osmonics, Minnetonka, MN) was also attached to the top side of the first layer by means of the pressure-sensitive silicone adhesive.

A second chip layer (including chamber top) having associated fluidic channels, ports, chambers, other reaction sites, etc. therein was cast in a mold using PDMS. This second layer was fashioned to be alignable with the first chip layer. The second layer was aligned with the chambers in the first chip layer and attached by means of the pressure-sensitive silicone adhesive, forming a completed chip. The PDMS top could function as a septum or a self-sealing membrane by itself, or in some cases, an additional partial layer of PDMS could be bonded over an inlet or outlet of the chip using the pressure-sensitive adhesive.

### Example 2

This example illustrates the preparation of a chip in accordance with an embodiment of the invention.

A chip layer having associated fluidic channels, ports, chambers, etc. therein was cast in polydimethylsiloxane (PDMS, Sylgard 184, Dow Coming, Midland, MI) using a machined aluminum mold. The PDMS layer was cured at 90 °C for 20 minutes. The PDMS layer was attached to a bottom plate by means of a pressure sensitive silicone adhesive layer (Dielectric Polymers, Holyoke, MA). The bottom plate was made of acrylic or polycarbonate and was machined from sheet stock or injection molded. The layers were bonded by compressing the layers in a hydraulic press (Carver, Wabash, IN), forming the completed chip. The PDMS top could function as a septum itself, or in some cases, an additional partial layer of PDMS could be bonded over an inlet or outlet of the chip using the pressure sensitive silicone adhesive.

### Example 3

This example illustrates the use of an embodiment of the invention to determine the turbidity of a solution. This example generally corresponds to the common practice of measuring cell density of bacterial cells by nephelometry (light scattering measured at 90° to the primary beam). See, generally, Methods for General Bacteriology, P. Gerhardt, Ed., 1981 Washington D.C. p. 197.

A chip having an integrated waveguide was constructed as follows. The top layer of the chip was prepared and cast with polydimethylsiloxane (PDMS, Sylgard 184, Dow Coming, Midland, MI) using a machined aluminum mold.

A short section of polymeric waveguide (500 microns square, acrylic; South Coast Fiber, Alachua, FL) was laid in the machined aluminum mold such that one end abutted the edge of the mold and the other end extended to the edge of the mold. Fluid PDMS was poured into the mold and allowed to cure. The PDMS was cured at 90 °C for 20 minutes, immobilizing the waveguide in the chip and creating a light path from the edge of the chip to a predetermined reaction site, a chamber. The cured PDMS layer was adhered to a flat polystyrene bottom layer, forming the completed chip (the PDMS layer spontaneously adhered to the polystyrene layer). The depth of the chamber form the surface of the chip was about 1 mm.

Light scattering was measured from a series of turbid solutions contained in the above chip. With reference to Fig. 3, the output of a helium-neon laser (05-LHP-991, wavelength = 632.8 nm; Melles Griot Lasers, Carlsbad, CA) was focused onto the end of waveguide 40 which transmitted the light to the reaction site 20. The detector 30 consisted of a collimating lens (74-UV f/2 lens; Ocean Optics, Dunedin, FL), an optical fiber (P600-2, 600 micron dia.; Ocean Optics), and an attached spectrophotometer (USB-2000F; Ocean Optics). The detection angle was ~90° from the axis of the waveguide.

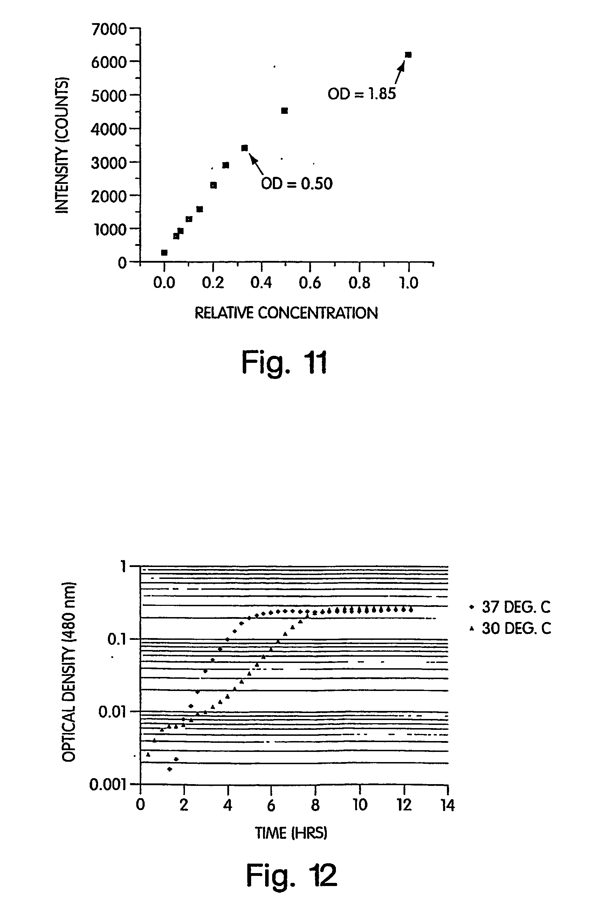

The reaction site was filled with a series of turbid solutions of non-dairy coffee creamer (Sugar Foods, New York, NY) which had absorbance values at 632 nm ranging from 0.05 to 1.85. A plot of scattered light intensity (632 nm) vs. relative concentration is given in Figure 11. Linear correlation was observed for the solutions with optical density values ranging from 0.05 to 0.5. At higher concentrations, the scattered light response became non-linear.

### Example 4

This example demonstrates an optically addressable reaction site, in accordance with an embodiment of the invention.

A chip was prepared using methods similar to those in Example 1. The chips used in this experiment were generally prepared. The distance of the reaction site from the surface of the chip was about 200 microns. As discussed below, the chip was optically addressed to measure optical density, using an arrangement similar to that pictured in Figure 1.

The light source (tungsten halogen, LH-1; Ocean Optics) was connected to an optical fiber (P100-2; Ocean Optics) which terminated with a collimating lens (74-UV; Ocean Optics) (not shown in Fig. 1). The optical fiber assembly delivered light 10 to a reaction site 20. The transmitted light 15, now at least partially attenuated by the turbidity of sample 25, was collected with another collimating lens/fiber assembly (not shown) which in turn transmitted to detector 30, a computer-controlled spectrophotometer (USB-2000; Ocean Optics). The optical density was calculated as OD = log(I/I₀).

The optical density ("OD") of a bacterial culture (E. coli BL21 in chemically defined media w/glucose) was monitored over a 13 hour growth period in a reaction site. The results from this experiment are shown in Fig. 12, which illustrates the growth of E. *coli BL21* at 30°C and 37 °C in the reaction sites of the chip, as monitored by a fiber optic spectrometer. These data thus demonstrate the validity of measuring cell growth by optically addressing reactions of the invention.

### Example 5

In this example, an embodiment of this experiment was used to demonstrate pH sensing.

Several chips similar to the one described in Example 1 were prepared. Each chip included a predetermined reaction site as defined by a chamber within the chip. The chamber depth of the bottom chamber (i.e., the distance of the chamber from the surface of the chip) was about 3 mm.

Fourteen solutions of 0.1 M phosphate buffer (K₂HPO₄/KH₂PO₄, both from Sigma-Aldrich, Milwaukee, WI) having differing pH were prepared with 5 micromolar solution of CDMF. CDMF (5(6)-carboxy-2',7'-dimethoxyfluorescein; Helix Research, Springfield, OR) is a fluorescent pH dye. A series of reaction sites on three different chips were each filled with the CDMF solutions.

The fluorescent intensity ("I") of the CDMF solutions in each chamber within each chip was measured upon excitation at two wavelengths, 510 nm and 450 nm. The light sources used for excitation were high intensity light-emitting diodes (LEDs, LXHL-BE01 and -BR02; Lumileds, San Jose, CA). The LED light was placed in optical communication with a 600 micron diameter optical fiber (P600-2, Ocean Optics) by a lens (74-UV, Ocean Optics), then directed to the chip. The emitted light was collected by a 25.4 mm f-1 lens (Thorlabs, Newton, NJ) and optically communicated to another 600 micron fiber which, in turn, was in optical communication with a computer-controlled spectrophotometer (LISB-2000F, Ocean Optics). The emission intensity reported in both cases was measured at 560 nm.

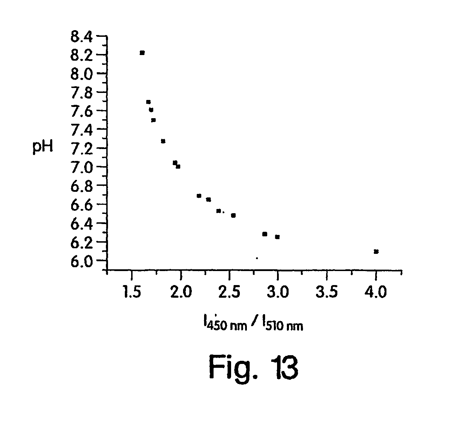

Sample results from these experiments are shown in Fig. 13, where the ratio of intensities was plotted versus the solution pH. Intensities were measured at 560 nm upon excitation by 450 nm light (I₄₅₀ₙₘ) and 510nm light (I₅₁₀ₙₘ), and the ratio of these values was plotted as (I_{450nm/}I₅₁₀ₙₘ). The response of the fluorescent signal was found to correlate well with pH over the range of at least about 6 to at least about 8.

Thus, this experiment demonstrating the capability of optically addressing one embodiment of the invention to measure and control pH using ratiometric fluorescence techniques.

While several embodiments of the invention have been described and illustrated herein, those of ordinary skill in the art will readily envision a variety of other means and structures for performing the functions and/or obtaining the results or advantages described herein, and each of such variations or modifications is deemed to be within the scope of the present invention. More generally, those skilled in the art would readily appreciate that all parameters, dimensions, materials, and configurations described herein are meant to be exemplary and that actual parameters, dimensions, materials, and configurations will depend upon specific applications for which the teachings of the present invention are used. Those skilled in the art will recognize, or be able to ascertain using no more than routine experimentation, many equivalents to the specific embodiments of the invention described herein. It is, therefore, to be understood that the foregoing embodiments are presented by way of example only and that, within the scope of the appended claims the invention may be practiced otherwise than as specifically described. The present invention is directed to each individual feature, system, material and/or method described herein. In addition, any combination of two or more such features, systems, materials and/or methods, if such features, systems, materials and/or methods are not mutually inconsistent, is included within the scope of the present invention.

In the claims (as well as in the specification above), all transitional phrases such as "comprising," "including," "carrying," "having," "containing," "involving," and the like are to be understood to be open-ended, i.e. to mean including but not limited to. Only the transitional phrases "consisting of" and "consisting essentially of' shall be closed or semi-closed transitional phrases, respectively, as set forth in the United States Patent Office Manual of Patent Examining Procedures, section 2111.03.

## Claims

1. A method comprising:
providing a polymeric chip comprising a plurality of reactors, each reactor comprising an inlet, an outlet, and a predetermined reaction site having a volume of less than 2 ml, the chip further comprising a semipermeable, substantially transparent membrane in contact with at least one predetermined reaction site, the membrane having a permeability to oxygen, and a permeability to water vapour that is at least one order of magnitude less than the permeability to oxygen, such that, along with other features, the reactor is constructed and arranged to maintain at least one living cell at the site and being suitable for cell culture;
causing a biological change in a biological material located at the predetermined reaction site; and
optically detecting the biological change in the biological material, and
wherein said method does not involve the use of human embryos.

2. The method of claim 1, wherein the chip is transparent.

3. The method of claim 1, wherein the chip comprises an inorganic material.

4. The method of claim 3, wherein the inorganic material comprises a semiconductor.

5. The method of claim 3, wherein the inorganic material comprises a metal.

6. The method of claim 1, wherein the living cell is a mammalian cell.

7. The method of claim 1, wherein the living cell is a bacterium.

8. The method of claim 1, wherein the living cell is pat of a tissue culture.

9. The method of claim 1, wherein the predetermined reaction site has a volume of less than 500 microlitres.

10. The method of claim 1, wherein the predetermined reaction site has a volume of less than 100 microlitres.

11. The method of claim 1, wherein the predetermined reaction site has a volume of less than 10 microlitres.

12. The method of claim 1, wherein the predetermined reaction site has a volume of less than 1 microlitre.

13. The method of claim 1, wherein the predetermined reaction site has a maximum dimension of less than 1 cm.

14. The method of claim 1, wherein the predetermined reaction site has a maximum dimension of less than 1 mm.

15. The method of claim 1, wherein the predetermined reaction site has a maximum dimension of less than 100 micrometers.

16. The method of claim 1, wherein the predetermined reaction site has a maximum dimension of less than 10 micrometers.

17. The method of claim 1, wherein at least one surface of the predetermined reaction site comprises a polymer.

18. The method of claim 17, wherein the at least one surface consists essentially of the polymer.

19. The method of claim 17, wherein the polymer is selected from the group consisting of a silicone, a polycarbonate, a polyethylene, a polypropylene, a polytetrafluoroethylene, a polyvinylidene chloride, a bis-benzocyclobutene, a polystyrene, a polyacrylate, a polymethacrylate, a polyimide and combinations thereof.

20. The method of claim 17, wherein the polymer is fluorinated.

21. The method of claim 1, wherein the reaction site has at least one substantially hydrophobic surface.

22. The method of claim 1, wherein the reaction site has at least one substantially hydrophilic surface.

23. The method of claim 1, wherein the reaction site has at least one substantially cytophilic surface.

24. The method of claim 1, wherein the reaction site has at least one substantially cytophobic surface.

25. The method of claim 21, 22, 23 or 24, further comprising at least one waveguide in optical communication with the predetermined reaction site.

26. The method of claim 1, further comprising a photodetector in optical communication with the predetermined reaction site.

27. The method of claim 26, wherein the photodetector is able to detect the presence of a cell at the predetermined reaction site.

28. The method of claim 27, wherein the cell is a mammalian cell.

29. The method of claim 27, wherein the photodetector is able to detect adhesion of the cell at the predetermined reaction site.

30. The method of claim 27, wherein the photodetector is able to detect a location of the cell at the predetermined site.

31. The method of claim 26, wherein the photodetector is in optical communication with a waveguide.

32. The method of claim 26, wherein the photodetector is able to detect light having a frequency of between 350 nm and 1000 nm.

33. The method of claim 26, wherein the photodetector comprises a photomultiplier.

34. The method of claim 26, wherein the photodetector comprises a photodiode.

35. The method of claim 1, further comprising a source of light in optical communication and integrally connected with the predetermined reaction site.

36. The method of claim 35, wherein the source of light comprises a light-emitting diode.

37. The method of claim 35, wherein the source of light comprises a laser.

38. The method of claim 37, wherein the laser comprises a semiconductor laser.

39. The method of claim 37, wherein the laser comprises a quantum well laser.

40. The method of claim 1, further comprising a filter able to filter light entering or exiting the predetermined reaction site, wherein the filter is integrally connected to the chip.

41. The method of claim 1, further comprising a light-interacting component integrally connected to the predetermined reaction site.

42. The method of claim 1, further comprising an actuator able to target a first cell type within the predetermined reaction site without targeting a second cell type.

43. The method of claim 1, wherein the reaction site has an inlet and an outlet.

44. The method of claim 1, further comprising at least one waveguide in optical communication with the predetermined reaction site.

45. The method of claim 44, wherein the waveguide is able to transmit visible light.

46. The method of claim 44, wherein the waveguide is able to transmit light having a wavelength of between 350 nm and 10,000 nm.

47. The method of claim 46, wherein the waveguide comprises a polymer.

48. The method of claim 47, wherein the waveguide consists essentially of the polymer.

49. The method of claim 47, wherein the waveguide comprises polystyrene, polyacrylate, polyinethacrylate, polycarbonate, polyimide, polyvinylidene fluoride, polyethylene, or polypropylene.

50. The method of claim 47, wherein the polymer is fluorinated.

51. The method of claim 47, wherein the polymer is a copolymer.

52. The method of claim 44, wherein the waveguide comprises a silicon-based material.

53. The method of claim 52, wherein the silicon-based material comprises glass.

54. The method of claim 52, wherein the silicon-based material comprises polysilicon.

55. The method of claim 52, wherein the silicon-based material comprises quartz.

56. The method of claim 52, wherein the silicon-based material comprises a carbide.

57. The method of claim 52, wherein the silicon-based material comprises a nitride.

58. The method of claim 52, wherein the silicon-based material comprises an oxide.

59. The method of claim 1, further comprising a milled or machined waveguide in optical communication with the predetermined reaction site.

60. The method of claim 1, further comprising an optical element in optical communication with the predetermined reaction site.

61. The method of claim 60, wherein the optical element is integrally connected to the apparatus.

62. The method of claim 60, wherein the optical element is a diffraction grating.

63. The method of claim 60, wherein the optical element is a lens.

64. The method of claim 63, wherein the lens is a diverging lens.

65. The method of claim 63, wherein the lens comprises a graded index material.

66. The method of claim 60, wherein the optical element is constructed and arranged to focus light on a waveguide.

67. The method of claim 60, wherein the optical element is constructed and arranged to focus light on a point located within the predetermined reaction site.

68. The method of claim 60, wherein the optical element is positioned so as to be able to focus light that will enter the predetermined reaction site.

69. The method of claim 60, wherein the optical element is positioned so as to collect light emitted from a point located within the predetermined reaction site.

70. A method, comprising:
providing a chip comprising a predetermined reaction site having an inlet, an outlet, and a volume of less that 2 ml, the predetermined reaction site further comprising a semipermeable, substantially transparent membrane in contact with the predetermined reaction site, the membrane having a permeability to oxygen, and a permeability to water vapour that is at least one order of magnitude less than the permeability to oxygen, such that, along with other features, the reactor is constructed and arranged to maintain at least one living cell at the site and being suitable for cell culture; and
optically addressing the predetermined reaction site; and
wherein said method does not involve the use of human embryos.

71. The method of claim 70, wherein optically addressing comprises optically addressing the predetermined reactions site using light that is in line-of-sight optical communication with the predetermined reaction site.

72. The method of claim 70, wherein optically addressing comprises optically addressing the predetermined reaction site using substantially monochromatic light.

73. The method of claim 70, wherein optically addressing comprises causing light-sensitive or light-activated reaction to occur.

74. The method of claim 70, further comprising:
providing material in the predetermined reaction site, the material having a smallest dimension;
directing electromagnetic radiation having an average beam diameter less than the smallest dimension of the material;
allowing the electromagnetic radiation to interact with the material to produce altered radiation; and
determining the altered radiation.

75. The method of claim 74, wherein the material comprises a cell.

76. The method of claim 74, wherein the determining step comprises quantifying the altered radiation.

77. The method of claim 74, wherein said method further comprises the step of determining a property of the interaction based on the measuring step.

78. The method of claim 74; wherein the measuring step comprises determining the optical density of the altered light.

79. The method of claim 74, wherein the electromagnetic radiation comprises visible light.

80. The method of claim 79, wherein the electromagnetic radiation consists essentially of visible light.

81. The method of claim 79, wherein the electromagnetic radiation is substantially monochromatic.

82. The method of claim 79, wherein the electromagnetic radiation has a wavelength of between 350 nm and 10,000 nm.

83. The method of claim 74, wherein the interaction comprises fluorescence.

84. The method of claim 74, wherein the interaction comprises light scattering.

85. The method of claim 70, further comprising optically causing a biological change in a biological material located at the predetermined reaction site.

86. The method of claim 85, wherein the biological material comprises a cell.

87. The method of claim 86, wherein the causing step comprises causing the cell to photosynthesize.

88. The method of claim 86, wherein the causing step comprises killing the cell.

89. The method of claim 86, wherein the cell is a plant cell.

90. The method of claim 70, further comprising providing material in the predetermined reaction site; directing light from a source within the chip at the material; and producing an image of the material.

## Patentansprüche

1. Methode umfassend:
das Bereitstellen eines polymeren Chip umfassend mehrere Reaktoren, wobei jeder Reaktor einen Einlass, einen Auslass und einen vorbestimmten Reaktionsort mit einem Volumen von weniger als 2 ml umfasst, wobei der Chip des Weiteren eine halbdurchlässige, im Wesentlichen transparente Membran umfasst, die mit mindestens einem vorbestimmten Reaktionsort in Kontakt steht, wobei die Membran eine Sauerstoffdurchlässigkeit sowie eine Durchlässigkeit für Wasserdampf aufweist, die um mindestens eine Größenordnung geringer ist als die Sauerstoffdurchlässigkeit, derart, dass der Reaktor zusammen mit anderen Merkmalen so konstruiert und angeordnet ist, um mindestens eine lebende Zelle, die für die Zellkultur geeignet ist, an dem Ort beizubehalten;
das Verursachen einer biologischen Änderung in einem biologischen Material, das sich an dem vorbestimmten Reaktionsort befindet; und
das optische Erfassen der biologischen Änderung des biologischen Materials, und
wobei die Methode nicht die Verwendung von menschlichen Embryos involviert.

2. Methode nach Anspruch 1, wobei der Chip transparent ist.

3. Methode nach Anspruch 1, wobei der Chip ein anorganisches Material umfasst.

4. Methode nach Anspruch 3, wobei das anorganische Material einen Halbleiter umfasst.

5. Methode nach Anspruch 3, wobei das anorganische Material ein Metall umfasst.

6. Methode nach Anspruch 1, wobei die lebende Zelle eine Säugerzelle ist.

7. Methode nach Anspruch 1, wobei die lebende Zelle eine Bakterie ist.

8. Methode nach Anspruch 1, wobei die lebende Zelle ein Häufchen Gewebekultur ist.

9. Methode nach Anspruch 1, wobei der vorbestimmte Reaktionsort ein Volumen von weniger als 500 Mikrolitern aufweist.

10. Methode nach Anspruch 1, wobei der vorbestimmte Reaktionsort ein Volumen von weniger als 100 Mikrolitern aufweist.

11. Methode nach Anspruch 1, wobei der vorbestimmte Reaktionsort ein Volumen von weniger als 10 Mikrolitern aufweist.

12. Methode nach Anspruch 1, wobei der vorbestimmte Reaktionsort ein Volumen von weniger als 1 Mikroliter aufweist.

13. Methode nach Anspruch 1, wobei der vorbestimmte Reaktionsort eine maximale Größe von weniger als 1 cm aufweist.

14. Methode nach Anspruch 1, wobei der vorbestimmte Reaktionsort eine maximale Größe von weniger als 1 mm aufweist.

15. Methode nach Anspruch 1, wobei der vorbestimmte Reaktionsort eine maximale Größe von weniger als 100 Mikrometern aufweist.

16. Methode nach Anspruch 1, wobei der vorbestimmte Reaktionsort eine maximale Größe von weniger als 10 Mikrometern aufweist.

17. Methode nach Anspruch 1, wobei mindestens eine Fläche des vorbestimmten Reaktionsorts ein Polymer umfasst.

18. Methode nach Anspruch 17, wobei die mindestens eine Fläche im Wesentlichen aus dem Polymer besteht.

19. Methode nach Anspruch 17, wobei das Polymer aus der Gruppe ausgewählt ist bestehend aus einem Silicon, einem Polycarbonat, einem Polyethylen, einem Polypropylen, einem Polytetrafluorethylen, einem Polyvinylidenchlorid, einem Bisbenzocyclobuten, einem Polystyrol, einem Polyacrylat, einem Polymethacrylat, einem Polyimid und Kombinationen derselben.

20. Methode nach Anspruch 17, wobei das Polymer fluoriert ist.

21. Methode nach Anspruch 1, wobei der Reaktionsort mindestens eine im Wesentlichen hydrophobe Fläche aufweist.

22. Methode nach Anspruch 1, wobei der Reaktionsort mindestens eine im Wesentlichen hydrophile Fläche aufweist.

23. Methode nach Anspruch 1, wobei der Reaktionsort mindestens eine im Wesentlichen zytophile Fläche aufweist.

24. Methode nach Anspruch 1, wobei der Reaktionsort mindestens eine im Wesentlichen zytophobe Fläche aufweist.

25. Methode nach Anspruch 21, 22, 23 oder 24, des Weiteren umfassend mindestens einen Wellenleiter in optischer Kommunikation mit dem vorbestimmten Reaktionsort.

26. Methode nach Anspruch 1, des Weiteren umfassend einen Photodetektor in optischer Kommunikation mit dem vorbestimmten Reaktionsort.

27. Methode nach Anspruch 26, wobei der Photodetektor dazu fähig ist, das Vorliegen einer Zelle an dem vorbestimmten Reaktionsort zu erfassen.

28. Methode nach Anspruch 27, wobei die Zelle eine Säugerzelle ist.

29. Methode nach Anspruch 27, wobei der Photodetektor dazu fähig ist, das Anhängen der Zelle an dem vorbestimmten Reaktionsort zu erfassen.

30. Methode nach Anspruch 27, wobei der Photodetektor dazu fähig ist, eine Position der Zelle an dem vorbestimmten Reaktionsort zu erfassen.

31. Methode nach Anspruch 26, wobei der Photodetektor in optischer Kommunikation mit einem Wellenleiter steht.

32. Methode nach Anspruch 26, wobei der Photodetektor dazu fähig ist, Licht mit einer Frequenz zwischen 350 nm und 1000 nm zu erfassen.

33. Methode nach Anspruch 26, wobei der Photodetektor einen Photomultiplizierer umfasst.

34. Methode nach Anspruch 26, wobei der Photodetektor eine Photodiode umfasst.

35. Methode nach Anspruch 1, des Weiteren umfassend eine Lichtquelle, die mit dem vorbestimmten Reaktionsort in optischer Kommunikation steht und integral damit verbunden ist.

36. Methode nach Anspruch 35, wobei die Lichtquelle eine lichtausstrahlende Diode umfasst.

37. Methode nach Anspruch 35, wobei die Lichtquelle einen Laser umfasst.

38. Methode nach Anspruch 37, wobei der Laser einen Halbleiterlaser umfasst.

39. Methode nach Anspruch 37, wobei der Laser einen Quantenwell-Laser umfasst.

40. Methode nach Anspruch 1, des Weiteren umfassend ein Filter, das dazu fähig ist, Licht zu filtrieren, das in den vorbestimmten Reaktionsort eintritt oder daraus austritt, wobei das Filter integral in den Chip eingebunden ist.

41. Methode nach Anspruch 1, des Weiteren umfassend eine mit Licht in Wechselwirkung stehende, integral in den vorbestimmten Reaktionsort eingebundene Komponente.

42. Methode nach Anspruch 1, des Weiteren umfassend ein Bedienungselement, das dazu fähig ist, einen ersten Zelltyp innerhalb des vorbestimmten Reaktionsorts anzuzielen, ohne einen zweiten Zelltyp anzuzielen.

43. Methode nach Anspruch 1, wobei der Reaktionsort einen Einlass und einen Auslass besitzt.

44. Methode nach Anspruch 1, des Weiteren umfassend mindestens einen Wellenleiter in optischer Kommunikation mit dem vorbestimmten Reaktionsort.

45. Methode nach Anspruch 44, wobei der Wellenleiter dazu fähig ist, sichtbares Licht zu transmittieren.

46. Methode nach Anspruch 44, wobei der Wellenleiter dazu fähig ist, Licht zu transmittieren, das eine Wellenlänge zwischen 350 nm und 10.000 nm besitzt.

47. Methode nach Anspruch 46, wobei der Wellenleiter ein Polymer umfasst.

48. Methode nach Anspruch 47, wobei der Wellenleiter im Wesentlichen aus dem Polymer besteht.

49. Methode nach Anspruch 47, wobei der Wellenleiter Polystyrol, Polyacrylat, Polymethacrylat, Polycarbonat, Polyimid, Polyvinylidenfluorid, Polyethylen oder Polypropylen umfasst.

50. Methode nach Anspruch 47, wobei das Polymer fluoriert ist.

51. Methode nach Anspruch 47, wobei das Polymer ein Copolymer ist.

52. Methode nach Anspruch 44, wobei der Wellenleiter ein Material auf Siliciumbasis umfasst.

53. Methode nach Anspruch 52, wobei das Material auf Siliciumbasis Glas umfasst.

54. Methode nach Anspruch 52, wobei das Material auf Siliciumbasis Polysilicium umfasst.

55. Methode nach Anspruch 52, wobei das Material auf Siliciumbasis Quarz umfasst.

56. Methode nach Anspruch 52, wobei das Material auf Siliciumbasis ein Carbid umfasst.

57. Methode nach Anspruch 52, wobei das Material auf Siliciumbasis ein Nitrid umfasst.

58. Methode nach Anspruch 52, wobei das Material auf Siliciumbasis ein Oxid umfasst.

59. Methode nach Anspruch 1, des Weiteren umfassend einen gefrästen oder maschinell bearbeiteten Wellenleiter in optischer Kommunikation mit dem vorbestimmten Reaktionsort.

60. Methode nach Anspruch 1, des Weiteren umfassend ein optisches Element in optischer Kommunikation mit dem vorbestimmten Reaktionsort.

61. Methode nach Anspruch 60, wobei das optische Element integral mit dem Apparat verbunden ist.

62. Methode nach Anspruch 60, wobei das optische Element ein Beugungsgitter ist.

63. Methode nach Anspruch 60, wobei das optische Element eine Linse ist.

64. Methode nach Anspruch 63, wobei die Linse eine divergierende Linse ist.

65. Methode nach Anspruch 63, wobei die Linse ein Gradientenindexmaterial umfasst.

66. Methode nach Anspruch 60, wobei das optische Element zum Fokussieren von Licht auf einen Wellenleiter konstruiert und angeordnet ist.

67. Methode nach Anspruch 60, wobei das optische Element zum Fokussieren von Licht auf einen Punkt konstruiert und angeordnet ist, der sich innerhalb des vorbestimmten Reaktionsorts befindet.

68. Methode nach Anspruch 60, wobei das optische Element so positioniert ist, dass es dazu fähig ist, Licht zu fokussieren, das in den vorbestimmten Reaktionsort eintritt.

69. Methode nach Anspruch 60, wobei das optische Element so positioniert ist, dass es Licht von einem Punkt auffängt, der sich innerhalb des vorbestimmten Reaktionsorts befindet.

70. Methode umfassend:
das Bereitstellen eines Chip umfassend einen vorbestimmten Reaktionsort mit einem Einlass, einem Auslass und einem Volumen von weniger als 2 ml, wobei der vorbestimmte Reaktionsort des Weiteren eine halbdurchlässige, im Wesentlichen transparente Membran umfasst, die mit dem vorbestimmten Reaktionsort in Kontakt steht, wobei die Membran eine Sauerstoffdurchlässigkeit sowie eine Durchlässigkeit für Wasserdampf aufweist, die um mindestens eine Größenordnung geringer ist als die Sauerstoffdurchlässigkeit, derart, dass der Reaktor zusammen mit anderen Merkmalen so konstruiert und angeordnet ist, um mindestens eine lebende Zelle, die für die Zellkultur geeignet ist, an dem Ort beizubehalten; und
das Anpeilen des vorbestimmten Reaktionsorts; und
wobei die Methode nicht die Verwendung von menschlichen Embryos involviert.

71. Methode nach Anspruch 70, wobei das optische Anpeilen das optische Anpeilen des vorbestimmten Reaktionsorts unter Anwendung von Licht umfasst, das sich in optischer Sichtlinienkommunikation mit dem vorbestimmten Reaktionsort befindet.

72. Methode nach Anspruch 70, wobei das optische Anpeilen das optische Anpeilen des vorbestimmten Reaktionsorts unter Anwendung von im Wesentlichen monochromatischem Licht umfasst.

73. Methode nach Anspruch 70, wobei das optische Anpeilen das Verursachen des Auftretens einer lichtempfindlichen oder lichtaktivierter Reaktion umfasst.

74. Methode nach Anspruch 70, des Weiteren umfassend:
das Bereitstellen von Material am vorbestimmten Reaktionsort, wobei das Material eine geringste Dimension aufweist;
das Richten elektromagnetischer Strahlung mit einem durchschnittlichen Strahldurchmesser von weniger als der geringsten Dimension des Materials;
das Erlauben, dass die elektromagnetische Strahlung mit dem Material unter Bildung einer geänderten Strahlung in Wechselwirkung tritt; und
das Bestimmen der geänderten Strahlung.

75. Methode nach Anspruch 74, wobei das Material eine Zelle umfasst.

76. Methode nach Anspruch 74, wobei der bestimmende Schritt ein Quantifizieren der geänderten Strahlung umfasst.

77. Methode nach Anspruch 74, wobei die Methode des Weiteren den Schritt des Bestimmens einer Eigenschaft der Wechselwirkung auf der Basis des Messschritts umfasst.

78. Methode nach Anspruch 74, wobei der Messschritt das Bestimmen der optischen Dichte des geänderten Lichts umfasst.

79. Methode nach Anspruch 74, wobei die elektromagnetische Strahlung sichtbares Licht umfasst.

80. Methode nach Anspruch 79, wobei die elektromagnetische Strahlung im Wesentlichen aus sichtbarem Licht besteht.

81. Methode nach Anspruch 79, wobei die elektromagnetische Strahlung im Wesentlichen monochromatisch ist.

82. Methode nach Anspruch 79, wobei die elektromagnetische Strahlung eine Wellenlänge zwischen 350 nm und 10.000 nm aufweist.

83. Methode nach Anspruch 74, wobei die Wechselwirkung Fluoreszenz umfasst.

84. Methode nach Anspruch 74, wobei die Wechselwirkung Lichtstreuung umfasst.

85. Methode nach Anspruch 70, des Weiteren umfassend das optische Verursachen einer biologischen Änderung in einem biologisch Material, das sich an dem vorbestimmten Reaktionsort befindet.

86. Methode nach Anspruch 85, wobei das biologische Material eine Zelle umfasst.

87. Methode nach Anspruch 86, wobei der verursachende Schritt das Verursachen, dass die Zelle photosynthetisiert wird, umfasst.

88. Methode nach Anspruch 86, wobei der verursachende Schritt das Abtötend der Zelle umfasst,

89. Methode nach Anspruch 86, wobei die Zelle eine Pflanzenzelle ist.

90. Methode nach Anspruch 70, des Weiteren umfassend das Bereitstellen von Material am vorbestimmten Reaktionsort; das Richten von Licht von einer Quelle innerhalb des Chip auf das Material; und das Bilden eines Bilds des Materials.

## Revendications

1. Méthode qui consiste à :
préparer une puce polymère qui comprend une pluralité de réacteurs, chaque réacteur comportant une entrée, une sortie et un site de réaction prédéterminé d'un volume inférieur à 2 ml, la puce comprenant également une membrane semiperméable en grande partie transparente qui est en contact avec au moins un site de réaction prédéterminé, et la membrane ayant une perméabilité à l'oxygène et une perméabilité à la vapeur d'eau qui est d'au moins un ordre de grandeur plus bas que la perméabilité à l'oxygène si bien qu'en conjonction avec d'autres caractéristiques, le réacteur est construit et configuré de façon à maintenir au moins une cellule vivante au site et à se prêter à la culture de cellules ;
causer une modification biologique dans un matériel biologique situé au site de réaction prédéterminé ; et
en option, détecter la modification biologique dans le matériel biologique,
ladite méthode ne faisant pas intervenir l'utilisation d'embryons humains.

2. Méthode de la revendication 1, où la puce est transparente.

3. Méthode de la revendication 1, où la puce comprend un matériau inorganique.

4. Méthode de la revendication 3, où le matériau inorganique comprend un semiconducteur.

5. Méthode de la revendication 3, où le matériau inorganique comprend un métal.

6. Méthode de la revendication 1, où la cellule vivante est une cellule mammifère.

7. Méthode de la revendication 1, où la cellule vivante est une bactérie.

8. Méthode de la revendication 1, où la cellule vivante fait partie d'une culture tissulaire.

9. Méthode de la revendication 1, où le site de réaction prédéterminé a volume inférieur à 500 microlitres.

10. Méthode de la revendication 1, où le site de réaction prédéterminé a volume inférieur à 100 microlitres.

11. Méthode de la revendication 1, où le site de réaction prédéterminé a volume inférieur à 10 microlitres.

12. Méthode de la revendication 1, où le site de réaction prédéterminé a volume inférieur à 1 microlitre.

13. Méthode de la revendication 1, où le site de réaction prédéterminé a une dimension maximale inférieure à 1 cm.

14. Méthode de la revendication 1, où le site de réaction prédéterminé a une dimension maximale inférieure à 1 mm.

15. Méthode de la revendication 1, où le site de réaction prédéterminé a une dimension maximale inférieure à 100 micromètres.

16. Méthode de la revendication 1, où le site de réaction prédéterminé a une dimension maximale inférieure à 10 micromètres.

17. Méthode de la revendication 1, où au moins une surface du site de réaction prédéterminé comprend un polymère.

18. Méthode de la revendication 17, où au moins une surface consiste essentiellement en le polymère.

19. Méthode de la revendication 17, où le polymère est sélectionné dans le groupe consistant en un silicone, un polycarbonate, un polyéthylène, un polypropylène, un polytétrafluoroéthylène, un chlorure de polyvinylidène, un bis-benzocyclobutène, un polystyrène, un polyacrylate, un polyméthacrylate, un polyimide et des combinaisons de ceux-ci.

20. Méthode de la revendication 17, où le polymère est fluoré.

21. Méthode de la revendication 1, où le site de réaction présente au moins une surface en grande partie hydrophobe.

22. Méthode de la revendication 1, où le site de réaction présente au moins une surface en grande partie hydrophile.

23. Méthode de la revendication 1, où le site de réaction présente au moins une surface en grande partie cytophile.

24. Méthode de la revendication 1, où le site de réaction présente au moins une surface en grande partie cytophobe.

25. Méthode de la revendication 21, 22, 23 ou 24, qui comprend également au moins un guide d'ondes en communication optique avec le site de réaction prédéterminé.

26. Méthode de la revendication 1, qui comprend également un photodétecteur en communication optique avec le site de réaction prédéterminé.

27. Méthode de la revendication 26, où le photodétecteur est capable de détecter la présence d'une cellule au site de réaction prédéterminé.

28. Méthode de la revendication 27, où la cellule est une cellule mammifère.

29. Méthode de la revendication 27, où le photodétecteur est capable de détecter l'adhésion de la cellule au site de réaction prédéterminé.

30. Méthode de la revendication 27, où le photodétecteur est capable de détecter l'emplacement de la cellule au site prédéterminé.

31. Méthode de la revendication 26, où le photodétecteur est en communication optique avec un guide d'ondes.

32. Méthode de la revendication 26, où le photodétecteur est capable de détecter une lumière dont la fréquence est comprise entre 350 nm et 1 000 nm.

33. Méthode de la revendication 26, où le photodétecteur comprend un photomultiplicateur.

34. Méthode de la revendication 26, où le photodétecteur comprend une photodiode.

35. Méthode de la revendication 1, qui comprend également une source de lumière en communication optique avec le site de réaction prédéterminé et intégralement connectée à celui-ci.

36. Méthode de la revendication 35, où la source de lumière comprend une diode émettrice de lumière.

37. Méthode de la revendication 35, où la source de lumière comprend un laser.

38. Méthode de la revendication 37, où le laser comprend un laser à semiconducteur.

39. Méthode de la revendication 37, où le laser comprend un laser à puits quantique.

40. Méthode de la revendication 1, qui comprend également un filtre capable de filtrer la lumière entrant dans le site de réaction prédéterminé ou en sortant, le filtre étant intégralement connecté à la puce.

41. Méthode de la revendication 1, qui comprend également un composant interagissant avec la lumière intégralement connecté au site de réaction prédéterminé.

42. Méthode de la revendication 1, qui comprend également un dispositif d'actionnement capable de cibler un premier type de cellules dans le site de réaction prédéterminé sans cibler un second type de cellules.

43. Méthode de la revendication 1, où le site de réaction présente une entrée et une sortie.

44. Méthode de la revendication 1, qui comprend également au moins un guide d'ondes en communication optique avec le site de réaction prédéterminé.

45. Méthode de la revendication 44, où le guide d'ondes est capable de transmettre la lumière visible.

46. Méthode de la revendication 44, où le guide d'ondes est capable de transmettre une lumière dont la longueur d'ondes est comprise entre 350 nm et 10 000 nm.

47. Méthode de la revendication 46, où le guide d'ondes comprend un polymère.

48. Méthode de la revendication 47, où le guide d'ondes consiste essentiellement en le polymère.

49. Méthode de la revendication 47, où le guide d'ondes comprend un polystyrène, polyacrylate, polyméthacrylate, polycarbonate, polyimide, polyfluorure de vinylidène, polyéthylène ou polypropylène.

50. Méthode de la revendication 47, où le polymère est fluoré.

51. Méthode de la revendication 47, où le polymère est un copolymère.

52. Méthode de la revendication 44, où le guide d'ondes comprend un matériau à base de silicium.

53. Méthode de la revendication 52, où le matériau à base de silicium comprend un verre.

54. Méthode de la revendication 52, où le matériau à base de silicium comprend un polysilicium.

55. Méthode de la revendication 52, où le matériau à base de silicium comprend un quartz.

56. Méthode de la revendication 52, où le matériau à base de silicium comprend un carbure.

57. Méthode de la revendication 52, où le matériau à base de silicium comprend un nitrure.

58. Méthode de la revendication 52, où le matériau à base de silicium comprend un oxyde.

59. Méthode de la revendication 1, qui comprend également un guide d'ondes fraisé ou usiné en communication optique avec le site de réaction prédéterminé.

60. Méthode de la revendication 1, qui comprend également un élément optique en communication optique avec le site de réaction prédéterminé.

61. Méthode de la revendication 60, où l'élément optique est intégralement connecté à l'appareil.

62. Méthode de la revendication 60, où l'élément optique est un réseau de diffraction.

63. Méthode de la revendication 60, où l'élément optique est une lentille.

64. Méthode de la revendication 63, où la lentille est une lentille divergente.

65. Méthode de la revendication 63, où la lentille comprend un matériau à gradient d'indice.

66. Méthode de la revendication 60, où l'élément optique est construit et configuré de façon à focaliser la lumière sur un guide d'ondes.

67. Méthode de la revendication 60, où l'élément optique est construit et configuré de façon à focaliser la lumière en un point situé dans le site de réaction prédéterminé.

68. Méthode de la revendication 60, où l'élément optique est positionné de façon à pouvoir focaliser la lumière qui entrera dans le site de réaction prédéterminé.

69. Méthode de la revendication 60, où l'élément optique est positionné de façon à recueillir la lumière émise d'un point situé dans le site de réaction prédéterminé.

70. Méthode, qui consiste à :
préparer une puce qui comprend un site de réaction prédéterminé doté d'une entrée et d'une sortie et dont le volume est inférieur à 2 ml, le site de réaction prédéterminé comprenant également une membrane semiperméable en grande partie transparente qui est en contact avec le site de réaction prédéterminé, et la membrane ayant une perméabilité à l'oxygène et une perméabilité à la vapeur d'eau qui est d'au moins un ordre de grandeur plus bas que la perméabilité à l'oxygène si bien qu'en conjonction avec d'autres caractéristiques, le réacteur est construit et configuré de façon à maintenir au moins une cellule vivante au site et à se prêter à la culture de cellules; et
réaliser l'adressage optique du site de réaction prédéterminé,
ladite méthode ne faisant pas intervenir l'utilisation d'embryons humains.

71. Méthode de la revendication 70, où la procédure d'adressage optique comprend un adressage optique du site de réaction prédéterminé au moyen d'une lumière qui en communication optique à visée directe avec le site de réaction prédéterminé.

72. Méthode de la revendication 70, où la procédure d'adressage optique comprend un adressage optique du site de réaction prédéterminé au moyen d'une lumière en grande partie monochromatique.

73. Méthode de la revendication 70, où la procédure d'adressage optique comprend le déclenchement d'une réaction sensible à la lumière ou activée par la lumière.

74. Méthode de la revendication 70, qui consiste également à :
transférer un matériel dans le site de réaction prédéterminé, le matériel ayant une dimension la plus faible ;
diriger un rayonnement électromagnétique dont le diamètre moyen du faisceau est inférieur à la dimension la plus faible du matériel ;
laisser le rayonnement électromagnétique interagir avec le matériel pour produire un rayonnement modifié ; et
déterminer le rayonnement modifié.

75. Méthode de la revendication 74, où le matériel comprend une cellule.

76. Méthode de la revendication 74, où l'étape de détermination comprend une quantification du rayonnement modifié.

77. Méthode de la revendication 74, où ladite méthode comprend également l'étape qui consiste à déterminer une propriété de l'interaction sur la base de l'étape de mesure.

78. Méthode de la revendication 74, où l'étape de mesure comprend la détermination de la densité optique de la lumière modifiée.

79. Méthode de la revendication 74, où le rayonnement électromagnétique comprend une lumière visible.

80. Méthode de la revendication 79, où le rayonnement électromagnétique consiste essentiellement en une lumière visible.

81. Méthode de la revendication 79, où le rayonnement électromagnétique est en grande partie monochromatique.

82. Méthode de la revendication 79, où le rayonnement électromagnétique a une longueur d'ondes comprise entre 350 nm et 10 000 nm.

83. Méthode de la revendication 74, où l'interaction comprend une fluorescence.

84. Méthode de la revendication 74, où l'interaction comprend une dispersion de la lumière.

85. Méthode de la revendication 70, qui comprend également le déclenchement, par un moyen optique, d'une modification biologique dans un matériel biologique situé au site de réaction prédéterminé.

86. Méthode de la revendication 85, où le matériel biologique comprend une cellule.

87. Méthode de la revendication 86, où l'étape de déclenchement entraîne une photosynthèse par la cellule.

88. Méthode de la revendication 86, où l'étape de déclenchement comprend la destruction de la cellule.

89. Méthode de la revendication 86, où la cellule est une cellule végétale.

90. Méthode de la revendication 70, qui consiste également à transférer un matériel dans le site de réaction prédéterminé ; à diriger, sur le matériel, une lumière émise d'une source située dans la puce; et à produire une image du matériel.
